# EUROPEAN PATENT APPLICATION

(11) **EP 2 813 254 A1**
(43) Date of publication of application: **17.12.2014**
(21) Application number: 13003022.4
(22) Date of filing: 13.06.2013
(51) Int. Cl.: A61M 5/14

(54) **Apparatus for simultaneous multiple medicine administration**

(71) Applicant: UMC Utrecht Holding B.V., 3584 CM Utrecht (NL)
(72) Inventor: Riphagen, Brechtje, 3512TX Utrecht (NL); Timmerman, Anna Monica Dori Egenie, 3521 DA Utrecht (NL); Jaspers, Joris Emanuel Nocilaas, 2411 AC Bodegraven (NL); Evers, Lucas Alphonsus Maria, 3707 CL Zeist (NL); Schoffelen, Richard Leonard Maria, 3533 AE Utrecht (NL); Mijers, Jan Willem Marinus, 2014 AL Haarlem (NL); van Dijk, Ronald, 3852 AG Ermelo (NL); van den Hoorn, Daniël, 3841 EJ Harderwijk (NL)
(74) Representative: Demski, Siegfried

(57) **Abstract**

The invention relates to an apparatus and a method for simultaneous multiple medicine administration. Apparatus and systems for simultaneous multiple medicine administration of prior art have the disadvantage of medicine flow rates being dependent on each other i.e. changing a particular flow rate leads to a change in the other flow rates. Moreover, the changed flow rate does not reach a set value in a short period of time but fluctuates around the set value for an undesired long period of time. This results in a wrong dosage of all medicines, if the flow rate of one medicine is adjusted, and can cause a serious damage to a patient since some medicine have a very narrow therapeutic range of safety.

The apparatus according to the invention eliminates this disadvantage and provides an almost exact dosage when administering multiple medicines simultaneously. The apparatus according to the invention comprises a housing in which at least two entry chambers and a collecting point are formed, wherein each entry chamber communicates with the collecting point via a flow path respectively and wherein in each flow path at least one penetration member is arranged, which causes a decrease in pressure downstream the penetration member with respect to the pressure upstream the penetration member. According to the method of the invention the independency of flow rates is eliminated irrespective of the equipment that is used, wherein a fluid is fed to a collecting point via separate flow paths and at least one penetration member is arranged within each flow path causing a decrease in pressure downstream the penetration member with respect to the pressure upstream the penetration member. The fluids eventually converge at the collecting point.

## Description

The invention relates to an apparatus for simultaneous administration of multiple medicines, comprising a housing, a plurality of inlets and one outlet.

When administering critical medicines, e.g. toxic ones or ones that can cause severe side effects, a precise dosage of said medicine is pursued in order to prevent an overdose that may interfere negatively and damage the patient's organism. The imprecisions of the apparatuses for administering multiple medicines known from the prior art lead to critical situations, which may even result in the patient's death. This situation is particularly perilous for premature infants and severely debilitated patients where the slightest derivation from the desired dosage may cause irreparable damages.

The apparatuses for administering multiple medicines contain several disadvantages. One of those disadvantages is the backflow of medicine. Once the pressure in one of the lines rises temporarily due to an altered volumetric flow of one of the medicines, the pressure rises in all lines connected to the apparatus. This results in a dosage of the other medicines which differ from the desired dosage. Furthermore, the medicine with the highest volumetric flow may run upstream into the direction of the pumps of the other medicaments, which is not intended. This leads to a temporary blockage of the volumetric flow of the other medicines. This causes a severe fault in the dosage of the designated medicines.

A known solution for this problem is the use of check valves in the inlets of the apparatus for administering multiple medicines from prior art. The flow of a medicine into another line connected to the apparatus is prevented. However, the volumetric flow of the medicine is blocked in the line with the shut check valve until the check valve is opened again. Therefore, the volumetric flow is temporarily disrupted, which leads to faults in the dosage of said medicine. Another disadvantage of those apparatuses for administering multiple medicines known from prior art is that the said apparatuses are located in the vicinity of the pumps for the individual medicines. Therefore, the medicines are brought together in a relative large distance from the patient and administered to them through one single line which may even be of an undesirable length. This causes a relatively large proportion of mixed medicaments in the line. While this is safe in a stable situation it can be hazardous in a situation in which the volumetric flow of the medicine has to be increased for the total volumetric flow of all medicines increases then and the higher volume of mixed medicines are administered in a shorter period of time than desired. All medicines are administered faster than they would have been if the volumetric flow of the single medicine had never been increased. This may also lead to dangerous situations.

A further disadvantage of those apparatuses for simultaneous administration of multiple medicines as known from the prior art is the interaction of medicines as a result of a chemical reaction between the individual medicines (e.g. crystallization, particle formation, increase of viscosity). This crystallization may lead to clogged lines. Also filters and check valves within the line may be clogged by the crystals.

A further disadvantage of prior art systems is that components are connected with tubing to form a multi port infusion set. These sets suffer from compliance due to the elasticity of the components, whereat each component is a possible source for air bubbles to entry into the system, which air bubbles are compressible. This leads to a dead volume being dependant on the pressure and thus on the pump settings.

An apparatus for administering multiple medicaments to a patient is known from US 3,941,126 A. The apparatus allows for an independent adjustment of the dissolution of the individual medicines. Here the dilutor is fed from a storage into a cylindrical container through opening valves, where the amount of the dilutor can be adjusted for each container. Subsequently, the medicine is added to the individual containers with the aid of syringes. By opening additional valves the diluted medicines flow in separate lines into a drip chamber where they are combined and mixed. The medicine then flows to a point of administration through another line.

US 2009/0137951 A1 discloses an apparatus for transferring several medicines into one line with several channels. The line ends in a connecting piece in the proximity of the patient, in which the medicines are brought together and mixed. The medicine mixture then flows from the connecting piece to an administration point. The medicines are brought together shortly before the administration point through the line that features several channels.

The invention is based on the task to provide an apparatus for administering multiple medicines to one administration point with a high precision while being able to adjust the volumetric flows of the individual medicines independently from each other.

In order to solve this task an apparatus is intended, which is characterized in that at least two entry chambers and a collecting point are formed within the housing which consists of a stiff material, wherein each entry chamber communicates with one inlet and is separated from the other entry chambers impervious to fluids and wherein the collecting point communicates with the outlet and in that the entry chambers communicate with the collecting point respectively via a flow path in which at least one penetration member is arranged causing a decrease in pressure downstream the penetration member with respect to the pressure upstream the penetration member. Other advantageous embodiments are described in the sub claims.

At least two fluid medicines are fed to the housing through separate lines. Each line is connected with one inlet of the housing which comprises at least two inlets, and every inlet communicates with one entry chamber respectively. After entering the entry chambers each fluid flows to the collecting point via a flow path respectively. In each flow path is at least one penetration member arranged. The penetration members dam up the individual medicines, so that the pressure downstream the penetration member is noticeably lower than the pressure upstream the penetration member. The medicines converge finally in the collecting point. The pressure in the collecting point is a little higher than the patient's blood pressure while typically much lower than the pressure in the lines feeding the medicines to the inlets formed on the housing. The pressure in the lines is caused by pumps which provide an adjustable volumetric flow of the medicines, wherein each line communicates with a pump. The volumetric flow is adjustable by means of the pumps. The volumetric flow of a particular medicine can thus be adjusted, if the dosage needs to be changed.

Another advantage of the inventive apparatus is that it is designed with a very small dead volume of the entry chambers and the flow paths, despite the integration of the penetration members within the housing. The housing consists of a stiff material imparting a very small elasticity to the housing. Moreover, the integration of all components in a single housing prevents the intrusion of air bubbles into the flow paths of the fluids. This leads to a constant and small dead volume of the apparatus according to the invention. The latter solves the problem of a changing dead volume when changing the pump settings. Thus, in the apparatus according to the invention the undesired compliance as described above is eliminated by arranging all the needed components in a single housing made of a stiff material to reduce the total compliance of the apparatus. The tubing material can also be chosen such that its compliance is minimized. Preferably, the housing consists of a stiff polymeric material, which can be transparent for visible light as well.

In conclusion, the very small dead volume of the apparatus as well as the decrease in pressure caused by the penetration members, which are each arranged within the flow paths, result in independent flow rates of the medicines, so that the flow rate of an individual medicine can be changed while ensuring a simultaneous stability of the flow rates of the other medicines. However, in reality the other flow rates are not completely stable when a big change in a flow rate occurs. A big change in a flow rate results in a corresponding short-termed change in the other flow rates. The change in the other flow rates, however, is very short-termed, which is why they re-reach their initial value after a short time. Therefore, the other flow rates show peaks with a very small half width in a diagram showing the flow rate over time. A strong increase of the flow rate of one medicine results in a short-termed increase of the flow rates of the other medicines. A strong decrease of the flow rate of one medicine results in a short-termed decrease of the flow rates of the other medicines. It is essential for the apparatus according to the invention that the changes in the non-manipulated flow rates are very short-lived, and the flow rates subsequently re-reach their initial value and keep it then without further fluctuations. With other infusion apparatus for simultaneous administration of multiple medicines known from prior art a change in the flow rate also results in a peak-like change of the other flow rates, however, they do not re-reach their initial value after a short period of time but oscillate around it for undesired long periods of time. This results in an uncertainty regarding the dosage of medicine administered to a patient in case a change in the flow rate of one or more medicines occurred during administration.

Furthermore, with the apparatus according to the invention a change in the flow rates results in a flow rate quickly approaching the set value and keeping it. In the apparatus known from prior art the adjustment of a certain flow rate results in a fluctuant approach to the set value, whereat the other flow rates fluctuate with a different amplitude as well. Therefore, the flow rate in the apparatus known from prior art reaches its set value only after undesired long periods (some more than one hour) and simultaneously interferes with the other flow rates.

A further effect with infusion apparatus of prior art is the so-called "overshoot" of a flow rate that was changed by means of a pump, which is characterized by the fact that the flow rate first reaches a value that is noticeably higher than the set value and subsequently falls down to the set value. This disadvantageous effect does not occur with the apparatus according to the invention. It is further especially advantageous that small, incremental changes in the flow rate do not affect the other flow rates, whereas, when employing prior art apparatus, even small changes in the flow rates lead to fluctuations in the other flow rates.

The corresponding dependence of the flow rates of different medicines in apparatus for simultaneous administration of multiple medicines known from prior art is highly relevant in practical medicine, since several medicines, such as inotropic, vasoactive, sedative and chemotherapy medicines, e.g., are toxic and are only to be administered to the patient in a well-defined amount. These types of drugs have a very narrow therapeutic range of safety, meaning that there is only little difference between toxic and effective therapeutic doses. An established balance is very critical and a small deviation in administered concentration can have significant clinical consequences. The well-defined administration, however, is not possible when changing one or more flow rates during the administration, which is why a patient receives a significantly different dosage than prescribed by the doctor in charge when using an apparatus known from prior art. The apparatus according to the invention eliminates those disadvantages as much as possible and therefore improves prior art noticeably and allows for a simultaneous administration of several medicines with almost exactly the dosage of each medicine as prescribed by the doctor in charge, whereat the dosage of one or several medicines can be changed without changing the dosage of the other medicines as well.

The penetration member can be a filter member, a membrane, an elastic element comprising at least one slit, a wall with at least one opening, a channel with a portion of decreased cross section area or an adjustable valve. These penetration members all cause a decrease in pressure in the direction of the flow, since they reduce the cross section area through which the medicines can flow and thereby damming up the medicines, so that the pressure downstream of a penetration member is significantly lower than the pressure upstream of the penetration member. The effect of the penetration member is that the liquid medicines are retained by the penetration member, so that a lower volume of a medicine flows through a penetration member per time unit than through the cross-section of a flow path immediately upstream of the penetration member, whereby the pressure downstream of the penetration member decreases. This lowered pressure then results, along with the small dead volume of the apparatus, in the fact that the flow rates of the medicines when converged do not influence each other, or, in case they do, only in a small amount and for a short period of time.

In a further embodiment of the apparatus according to the invention it can be intended for two or more penetration members to be consecutively arranged within one or more flow paths respectively, such that a fluid flowing through a flow path passes one penetration member after another within one flow path. With this embodiment one or more penetration members are arranged in a row within a flow path, so that the pressure drops in sections. It can be intended to arrange one or more penetration members of the same kind or different penetration members within the flow path. Thus, it can be made use of different characteristics of different penetration members.

It is possible to arrange a penetration member in the form of a filter member and another penetration member, e.g. in the form of a channel with a portion of decreased cross section area, between the collecting point and one respective entry chamber within one or more flow paths. The filter member results both in a drop in pressure and filters solid matters from the fluid medicine. The penetration members arranged within the flow path can be chosen depending on the characteristics of the medicine administered or due to the expenditure in production.

The flow paths between the collecting point and the entry chambers can be formed as flow channels with a small cross-section area. By forming the flow paths as flow channels the dead volume, which also influences the independence of the flow rates can be designed very small. The apparatus is not limited to simple geometries, however, the flow paths can feature a complex geometry as well.

In a further embodiment of the invention it can be intended for the collecting point within the housing of the apparatus according to the invention to be formed as a collecting chamber. In this embodiment the medicines are brought together in the collecting chamber, whereat the volume of the collecting chamber can result in another drop in pressure, if the medicines are fed to the collecting chamber via flow paths with a small cross-section area. The pressure within the collecting chamber is then slightly higher than the blood pressure of a patient, so that the mixed medicines flow into the patient's bloodstream.

The apparatus according to the invention can be designed in such a way that at least one penetration member is a filter member which consists of two or more filter elements, wherein each filter element is arranged in one flow path respectively. As mentioned above, a filter member both causes a drop in pressure and prevents solid matters from entering the patient's bloodstream. Nevertheless, it may be necessary to filter the medicines in the apparatus with filter members of different characteristics. Filter members may be distinguished by their pore size, by additives applied to the surface or by different electric charges. Instead of arranging separate filter members within two or more flow path a filter member which consists of several filter elements can be arranged within the apparatus in this advantageous embodiment. Said filter member is arranged in such a way in the housing that each filter element is located in one flow path respectively. A filter member consisting of several filter elements therefore simplifies the manufacture of the apparatus according to the invention and thus reduces manufacturing costs. Two or more filter elements may consist of different materials and/or two or more filter elements may have different meshes, pore sizes or electrical charges. Furthermore, the filter member can be provided with an additional anti-bacterial substance applied to the filter surface, or the filter elements can be provided with the same or different additional antibacterial substances.

The outlet of the apparatus can be arranged centrically at the housing when filter elements of the same filter surface are used. In case of filter elements with different filter surfaces the outlet can also be arranged at any other position of the housing. The latter may become necessary for administering medicines with very different flow rates to a patient by the apparatus according to the invention, whereat one flow rate may be significantly higher than the others.

It can also be intended for at least one penetration member to be a filter member which is preassembled in a filter frame by joining, molding, glueing, ultra sonic welding or heat sealing, wherein the filter frame is fixed on at least one inner surface of the housing. With this embodiment of the apparatus according to the invention said apparatus can be manufactured easily, since the filter member is not subject to the dangers of damages during installation, but is already pre-installed in a frame which is arranged in the housing of the apparatus and fixed to an inner surface of the housing.

The apparatus according to the invention may furthermore be designed in such a way that the entry chambers are separated from each other by walls protruding from at least one inner surface of the housing and/or by walls protruding from at least one penetration member arranged within one or more entry chambers or in the vicinity thereof. The entry chambers can be separated from each other by walls protruding from at least one inner surface of the housing, whereat the walls come in contact with an opposite inner surface of the housing or other walls extending from an opposite inner surface of the housing when assembling the housing, so that the entry chambers are separated from each other impervious to fluids. However, it can also be intended for a penetration member to be arranged in or in the vicinity of one or more entry chambers, wherein walls protrude from said penetration member, whose walls come in contact with the inner surfaces of the housing when assembling the housing and thus separate the entry chambers from each other impervious to fluids. For instance, a filter member preassembled in a filter frame can be arranged within the housing in such a way that the entry chambers are separated from each other by the walls protruding from the filter frame.

In a further embodiment of the apparatus according to the invention the housing may comprise ribs extending from at least one inner surface of the housing into the entry chambers, which provide for a planar flow of the medicines within the entry chambers. Those ribs prevent the development of a turbulent flow within the entry chambers. In case a filter member is arranged within one or several entry chambers the ribs also serve as support members for the filter surface, since the drop in the pressure downstream of the filter member caused by the filter member could result in a deflection of the filter surface.

Moreover, it can be intended for one or more entry chambers to communicate with one inlet respectively via a flow channel with a decreased cross-section area with respect to the cross-section of the inlet. The flow channel causes an advantageous small storage of the medicines fed to each inlet before they enter the entry chambers.

In order to eliminate a back-flow of medicines within the housing of the apparatus according to the invention a check valve is positioned within at least one flow path, within at least one entry chamber or within at least one inlet. Even though the drop in pressure between the entry chambers and the collecting point already prevents a back-flow of medicines within the housing a prevention of a back-flow can be ensured by arranging a check valve within at least one flow path, within at least one entry chamber or within at least one inlet. A back-flow of medicines would cause a contamination of the other flow paths within the housing which in turn results in an uncertainty of the real dosage of an administered medicine. Moreover, the back-flow of a medicine beyond the inlets may cause a contamination of the pumps feeding the medicines to the inlets. Such a contamination entails a complicated cleaning of the affected pumps.

In a further advantageous embodiment of the apparatus according to the invention at least one bore is formed on the housing, wherein the bore is covered by a hydrophobic filter thereby allowing for air/gas bubbles in the fluid medicines fed to the entry chambers to escape from the fluids. Air bubbles within the medicine are always undesirable, since they must not reach the patient's bloodstream. Still, in reality gas bubbles, in particular air bubbles, can result from the conveyance of a medicine from the storage vessel to the inlets of the apparatus. These gas bubbles can gather at the entry chambers of the housing, in particular when a hydrophilic filter member is arranged within the housing, and result in a blockage of the medicine flow. The at least one bore is therefore preferably formed on the housing in such a way that it communicates with an entry chamber and is covered by a hydrophobic filter, allowing for the gas bubbles to escape from the entry chamber.

Furthermore, it can be intended for at least one penetration member to be fixed to an inner surface of the housing by joining, molding, glueing, ultrasonic welding or heat sealing. When choosing the kind of fixation the materials of the penetration member, the material of the inner surface and the manufacturing costs are considered.

The apparatus according to the invention can be designed in such a way that the inlets are arranged on a circumference line of the housing which circumference line has a diameter of 1 mm to 40 mm. In this embodiment of the apparatus the housing features a flat form and a size that is easy to handle. Due to the flat form of the housing it may, e.g., be put on a lying patient and thus be in immediate approximation of the infusion point. Having the apparatus near the infusion point is advantageous independently of the form of the housing, since the medicines converge at the collecting point and flow then to the infusion point through a common line. The longer the common line is, the bigger is the mixing of the medicines. A mixture of the medicines outside the bloodstream is generally undesired, since the medicines may interact (crystallization, incompatibility between medicines).

Furthermore the collection chamber can have a volume of 0.1 ml to 1 ml. As mentioned above a small dead volume of the apparatus according to the invention is a factor for the independence of the flow rates of the medicines, which are fed to the apparatus. By designing the collecting point as a collecting chamber another drop in pressure may occur. The pressure in the collecting chamber is then a little higher than the patient's blood pressure. A volume of a collecting chamber of 0.01 ml to 1 ml is therefore a compromise which benefits both effects.

In a further embodiment of the apparatus according to the invention it can be intended for at least one inlet to be designed to be rotatable. Due to the rotatable design of at least one inlet the handling of the apparatus is simplified, since the lines feeding the fluid medicines to the apparatus can be arranged and placed more easily, e.g. parallel to the apparatus.

The housing of the apparatus according to the invention can advantageously consist of one or more housing elements, wherein the housing elements are welded, press fitted, glued and/or snap fitted with each other. Depending on the concrete geometric design of the housing of the apparatus according to the invention it can consist of two or more housing elements, which can be individually produced in an easy and cost-effective way and subsequently be connected with each other.

The invention further relates to a method for a simultaneous multiple medicament administration, wherein the medicines are fluids, characterized in that the fluids are fed to a collecting point via separate flow paths, and at least one penetration member is arranged within each flow path causing a decrease in pressure downstream the penetration member with respect to the pressure upstream the penetration member, wherein the fluids converge at the collecting point.

According to-this method the medicines are fed to a collecting point through separate flow paths, wherein at least one penetration member is arranged in each flow path. The penetration members cause a drop in the pressure in the respective flow path. In case only one penetration member is arranged in each flow path the drop of the pressure results in a pressure that is only a little higher than the patient's blood pressure. The flow paths communicate the collecting point in which the medicines converge. The lengths of the flow paths are irrelevant, but should be formed as short as possible in order to minimize the dead volume. Therefore, the flow paths may feature an extension in flow direction or both the collecting point and the entry chambers about the respective penetration members such that the collecting point and the entry chambers are separated only by the respective penetration member. The latter may be the case when a single penetration member with a flat prolonged shape, such as a filter member, is arranged between the entry chambers and the collecting point, which is then formed as a collecting chamber.

The penetration members arranged in the flow paths can be a filter member, a membrane, an elastic member comprising at least one slit, a wall with at least one opening, a channel with a portion of a decreased cross-section area or an adjustable valve.

In each flow path respectively two or more penetration members can be arranged consecutively, such that a fluid flowing through a flow path passes one penetration member after another. In this embodiment of the method according to the invention the pressure drops in sections within the flow path to the pressure of the collecting point. The pressure at the collecting point is preferably a little higher than the patient's blood pressure. The reduction of the pressure within the flow paths and a small dead volume of the flow paths result in the independence of the flow rates of the fluid medicines when those converge at the collecting point.

The invention is further explained in detail with the aid of the figures, whereat
- Fig. 1: shows a perspective view of a first embodiment of an apparatus according to the invention,
- Fig. 2: shows an exploded view of the first embodiment,
- Fig. 3: shows a perspective bottom view of the second housing element,
- Fig. 4: shows a perspective bottom view of the first housing element,
- Fig. 5: shows a perspective view of an adapter,
- Fig. 6: shows a perspective bottom view of an adapter,
- Fig. 7: shows a perspective top view of a check valve,
- Fig. 8: shows a further perspective view of the check valve,
- Fig. 9: shows a perspective and partly sectional view of the first embodiment,
- Fig. 10: shows the first housing element of a second embodiment of the apparatus according to the invention,
- Fig. 11: shows a third embodiment of the apparatus according to the invention,
- Fig. 12: shows an exploded view of the third embodiment,
- Fig. 13: shows a further exploded view of the third embodiment,
- Fig. 14: shows a sectional view of the second housing element of the third embodiment,
- Fig. 15: shows a perspective bottom view of the fourth housing element of the third embodiment,
- Fig. 16: shows a sectional view of the fourth housing element of the third embodiment,
- Fig. 17: shows a perspective view of the third housing element of the third embodiment,
- Fig. 18: shows a perspective view of the first housing element of the third embodiment,
- Fig. 19: shows a sectional view of the first housing element of the third embodiment,
- Fig. 20: shows a sectional view of the third embodiment,
- Fig. 21: shows a fourth embodiment of an apparatus according to the invention
- Fig. 22: shows a perspective bottom view of the first housing element of the fourth embodiment
- Fig. 23: shows a cut view of the first housing element of the fourth embodiment,
- Fig. 24: the third housing element of the fourth embodiment,
- Fig. 25: shows a perspective bottom view of the third housing element of the fourth embodiment
- Fig. 26: shows a perspective top view on the third housing element of the fourth embodiment
- Fig. 27: shows a sectional view of the fourth embodiment,
- Fig. 28: shows a second embodiment of the fourth housing element,
- Fig. 29: shows a sectional view of the second embodiment of the fourth housing element,
- Fig. 30: a second embodiment of the second housing element,
- Fig. 31: shows a sectional view of the second embodiment of the second housing element,
- Fig. 32: shows a sectional view of the second embodiment of the second housing element and the fourth housing element,
- Fig. 33: shows the result of a measurement of the flow rates of three medicines as a function of time, whereat the three medicines converge by means of an apparatus as known from prior art
- Fig. 34: shows a measurement curve of flow rates as recorded by the apparatus according to the invention and
- Fig. 35: shows a table summarizing the basic advantages of the apparatus according to the invention.

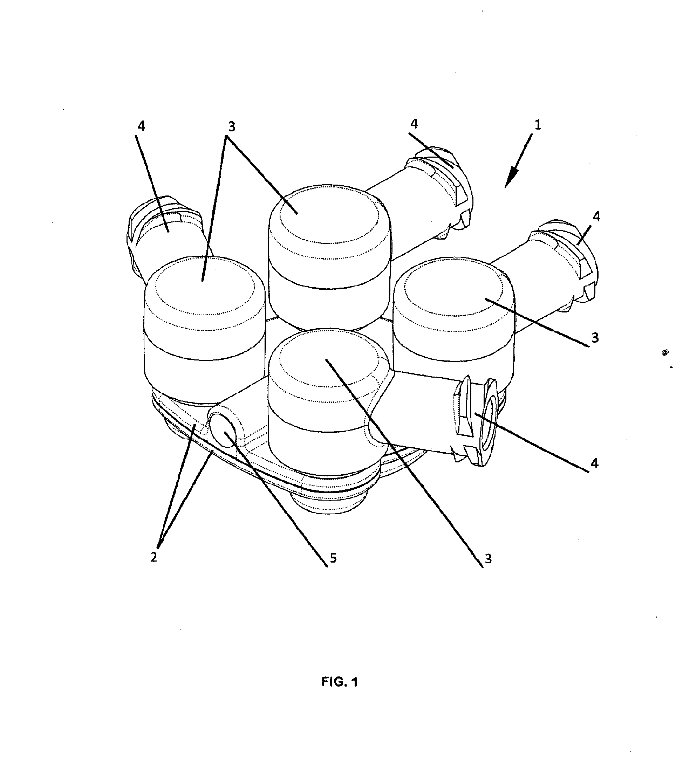

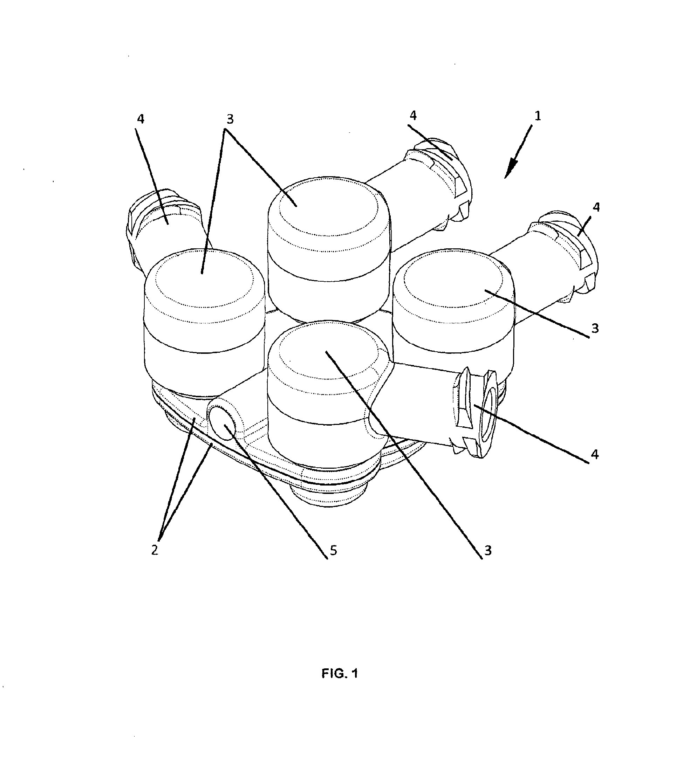

Figure 1 shows a perspective view of a first embodiment of an apparatus 1 according to the invention. Apparatus 1 consists of a housing 2 onto which adapters 3 are arranged. Fluid medicines are fed through the adapters 3 to the housing. For an easy connection of feed lines to the apparatus 1 a Luer-Lock-Connector 4 is arranged to each adapter 3, allowing for the feed lines to be connected needle-freely to the housing 2. The liquids combined in the housing 2 exit the housing through an outlet 5 and are fed to the patient through a common line.

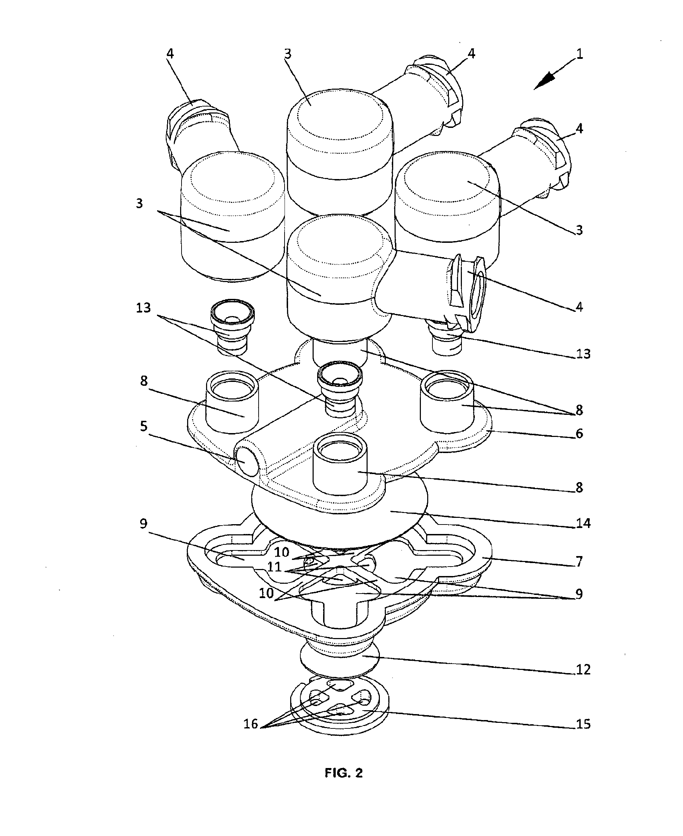

Figure 2 shows an exploded view of the embodiment of an apparatus 1 according to the invention. The housing 2 of the apparatus 1 comprises a first housing element 6 and a second housing element 7. The first housing element 6 features four stepped inlets 8, whereat each inlet 8 communicates with an entry chamber 9. The entry chambers 9 are separated imperviously to fluids through walls 10. A check valve 13 is arranged in each inlet 8, so as to prevent a backflow of fluids to the feed lines from the entry chambers 9. Each inlet 8 features an adapter 3, preferably formed in one piece. A Luer-Lock-Connector 4 is formed on each adapter 3. The adapters 3 are arranged in such a way that they are rotatable at the inlets 8. Between the first housing element 6 and the second housing element 7 a hydrophilic filter member 14 is arranged and is in contact with the walls 10. The hydrophilic filter member 14 is connected edgesided with the housing, e.g. by gluing or welding. The housing furthermore features bores 11, which are covered by a hydrophobic filter 12. The hydrophobic filter 12 is pressed to the second housing element 7 by a third housing element 15, so that an impermeable connection occurs between the hydrophobic filter 12 and the second housing element 7. The third housing element 15 features bores 16 allowing gases, in particular air, penetrating the hydrophobic filter to escape. The bores 11, 16 prevent together with the hydrophobic filter 12 that gas is gathered in the entry chambers 9, which could cause a blockage of the medicine flow.

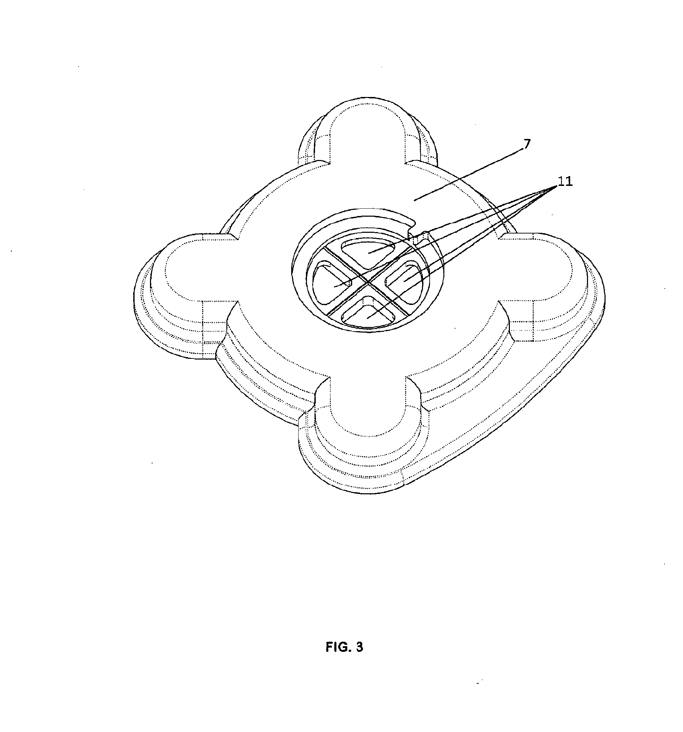

Figure 3 shows a perspective bottom view of the second housing element 7 with the bores 11. The hydrophobic filter 12 (not depicted) is arranged between the second housing element 7 and the third housing element 15 (not depicted), which offers the possibility of escape for gas bubbles.

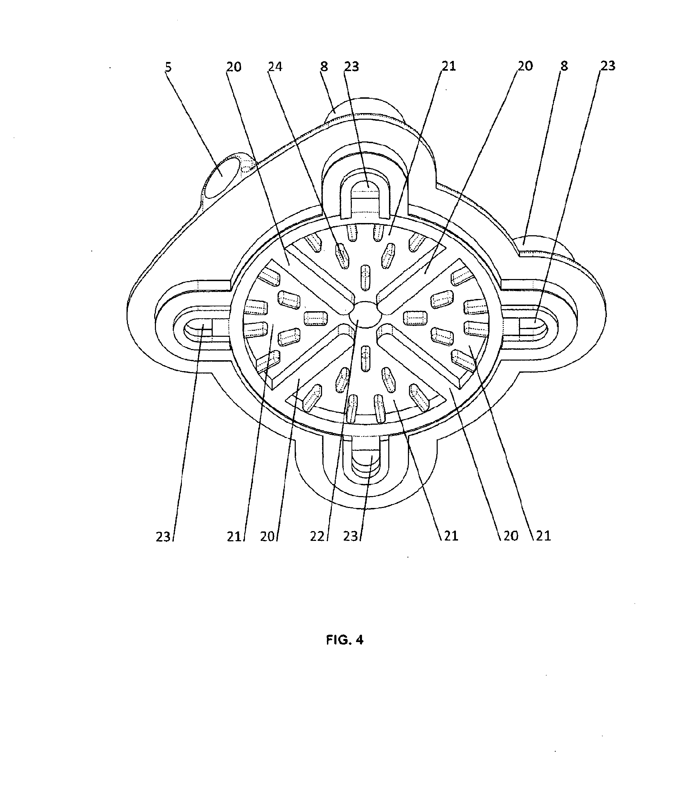

Figure 4 shows a perspective bottom view of the first housing element 6. The inlets 8 each feature a flow channel 23, which communicates with an entry chamber 9 respectively. Walls 20 extend from an inner surface of the first housing element 6 bordering regions 21. The fluids fed to the entry chambers 9 pass the hydrophilic filter member 14 and enter the regions 21. The walls 20 are arranged directly above the walls 10 of the second housing element 7. The hydrophilic filter member 14 is arranged between the walls 10 of the second housing element and the walls 20 of the first housing element. The hydrophilic filter member 14 is trapped between the walls 10 and the walls 20, so that the entry chambers 9 are separated from each other impervious to fluids. The first housing element 6 further comprises a collecting point 22 at which the fluids converge. The walls 20 ensure a separation of the fluids fed to the entry chambers 9 and passing the hydrophilic filter member 14 until they reach the collecting point 22 to prevent an unwanted mixture of the fluids. The inner surface of the first housing element 6 further features elevations 24 in the form of ribs extending radially in the regions 21. The elevations 24 serve two functions. On the first hand the elevations 24 serve as a support for the hydrophilic filter member 14 arranged between the first housing element 6 and the second housing element 7. On the other hand the elevations cause a planar flow of the fluids passing through the hydrophilic filter, due to their elongated form into the direction of the collecting point 22.

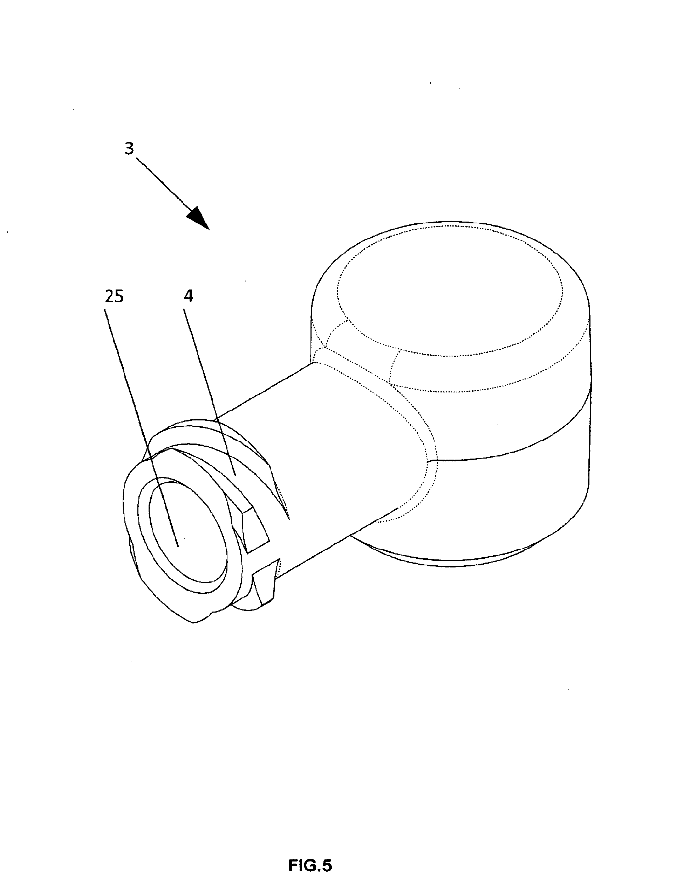

Figure 5 shows a perspective view of an adapter 3. The adapter 3 features an angled flow channel 25 and a Luer-Lock-Connector 4.

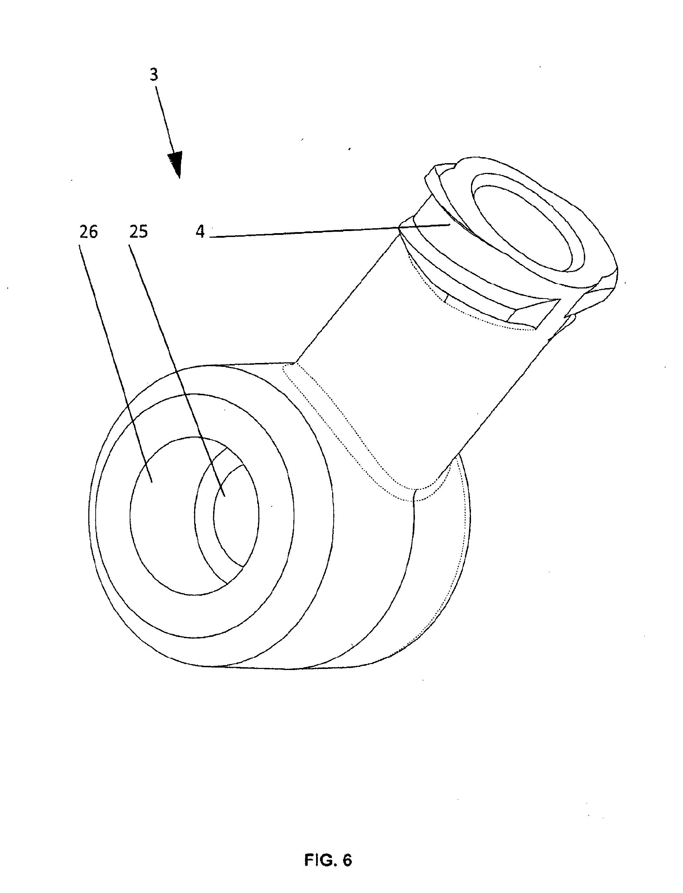

Figure 6 shows a perspective bottom view of an adapter 3. In this figure the downstream end of the angled flow channel 25 can be seen. The adapter 3 features a seat 26 at its end section facing the housing 2 of the apparatus according to the invention. Said seat 26 comes in a form-fitted contact with the outer surface of an inlet 8. Due to its design the end section of an adapter 3 can be connected to an inlet impervious to fluids, whereat the adapter can be rotated after connection with an inlet 8. The connection of the adapter 3 to the inlet 8 can also occur by means of a press-fit or clip fastening.

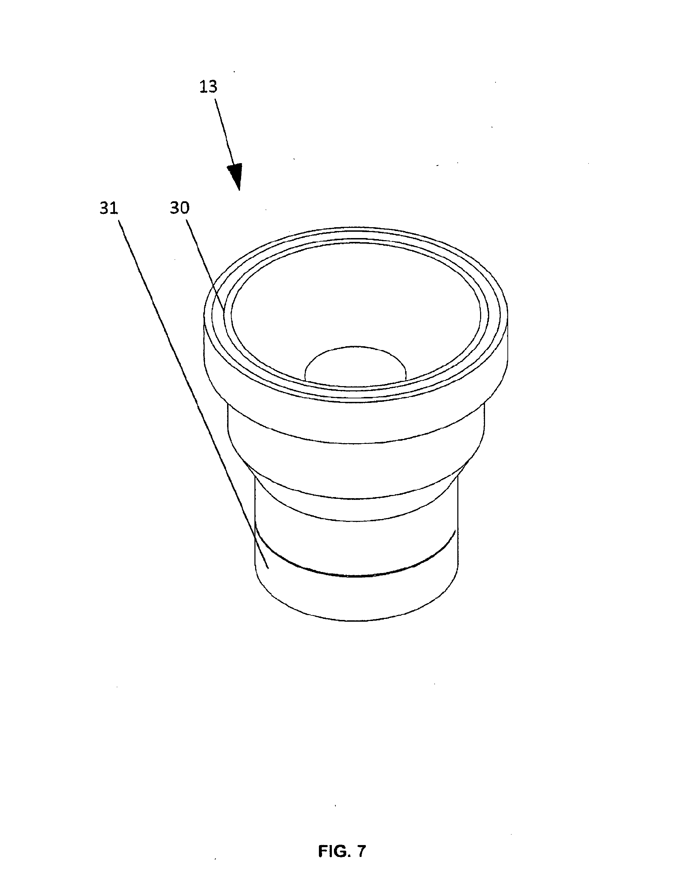

Figure 7 shows a perspective top view of a check valve 13. The check valve 13 features two end sections 30, 31 and is designed cylindrically. The end section 30 features a bigger diameter than the end section 31. The check valve 13 is arranged in that a way that it protrudes forward into the inlet 8 with its end section 31, whereat its end section 30 rests on a nose formed in the inlet 8.

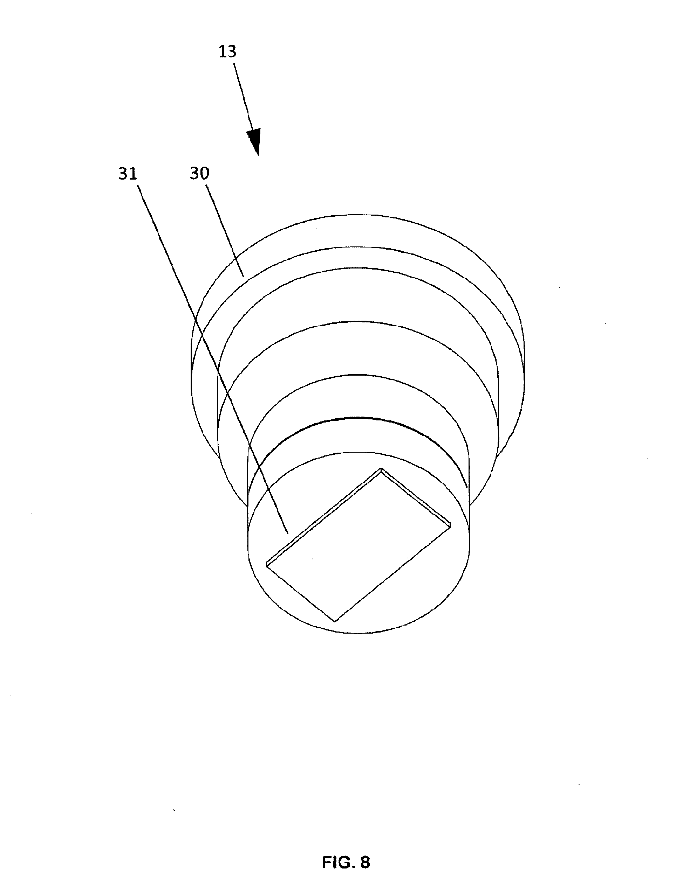

Figure 8 shows a further perspective view of the check valve 13. It is apparent that the end section 30 has a bigger diameter than the end section 31. Particularly the end section 30 features a protruding edge. Due to the protruding edge the check valve 13 can rest on the nose formed in an inlet 8. The end section 31 protrudes into an inlet 8.

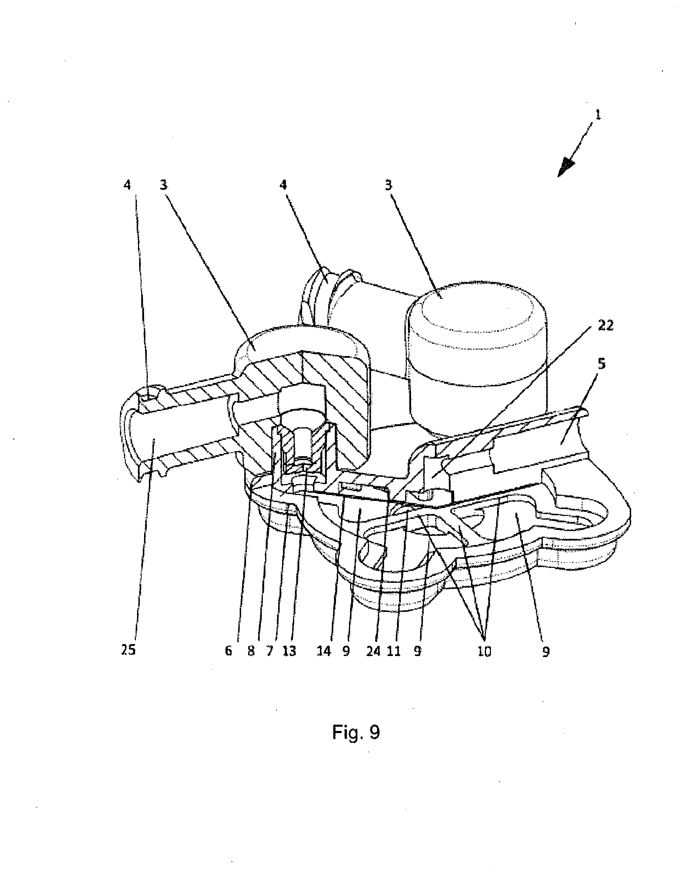

Figure 9 shows a perspective and partly sectional view of the first embodiment of the apparatus 1 according to the invention, whereat the first housing element 6 and an adapter 3 are depicted in a sectional view. A fluid fed to the apparatus through a line enters the angled flow channel 25 and flows through the check valve 13 arranged in an inlet 8 into an entry chamber 9. Each entry chamber 9 is separated from the other entry chambers 9 impervious to fluids by walls 10 extending from one inner surface of the second housing element 7. The inner surface of the second housing element 7 features bores 11. A hydrophobic filter is arranged underneath those bores 11, which allow gas bubbles in the fluid to escape the housing. The fluid then passes the hydrophilic filter member 14 and flows into a region 21 downstream of the hydrophilic filter member 14. The fluid flows then to the collecting point 22 within the area 21, whereat it remains separated from the fluids fed to the other entry chambers 9. The collecting point 22 is formed in the center of the inner surface of the first housing element 6, from which the walls 20 extend. The fluid converges with the other fluids fed to the apparatus at the collecting point 22. The fluids converge at the collecting point 22 and flow then into the outlet of the apparatus 1.

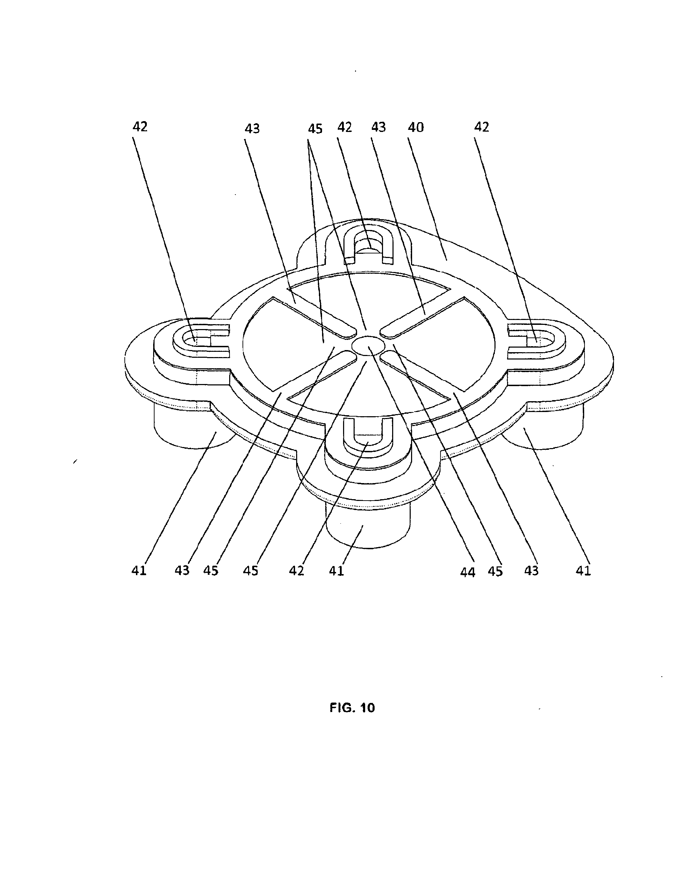

Figure 10 shows the first housing element 40 of a second embodiment of the apparatus according to the invention. The second housing element 40 is identical to that of the first embodiment. Like the first housing element 6 of the first embodiment the first housing element 40 comprises four inlets 41 with flow channels 42 which each lead to an entry chamber. Walls 43 extend from an inner surface of the first housing element 40. Said walls 43 are arranged directly above walls extending from an inner surface of the second housing element. In contrast to the first embodiment, the regions between the walls 43 are parts of the entry chambers 9, for there is no hydrophilic filter member arranged between the first housing element 40 and the second housing element of the second embodiment, in contrast to the first embodiment. Between the end sections of the walls 43 facing the collecting point 44 there are gaps 45, through which the fluids flow from the entry chambers 9 to the collecting point 44. Those small gaps 45 cause the fluids to be retained in the entry chambers 9 and consequently a drop in pressure at the collecting point 44 occurs. The gaps 45 are the only penetration members arranged in the apparatus according to this embodiment. The flow path between the entry chambers 9 and the collecting point 44 is designed very short in this embodiment, since the entry chambers 9 communicate with the collecting point 44 only by the gaps 45 between the walls 43.

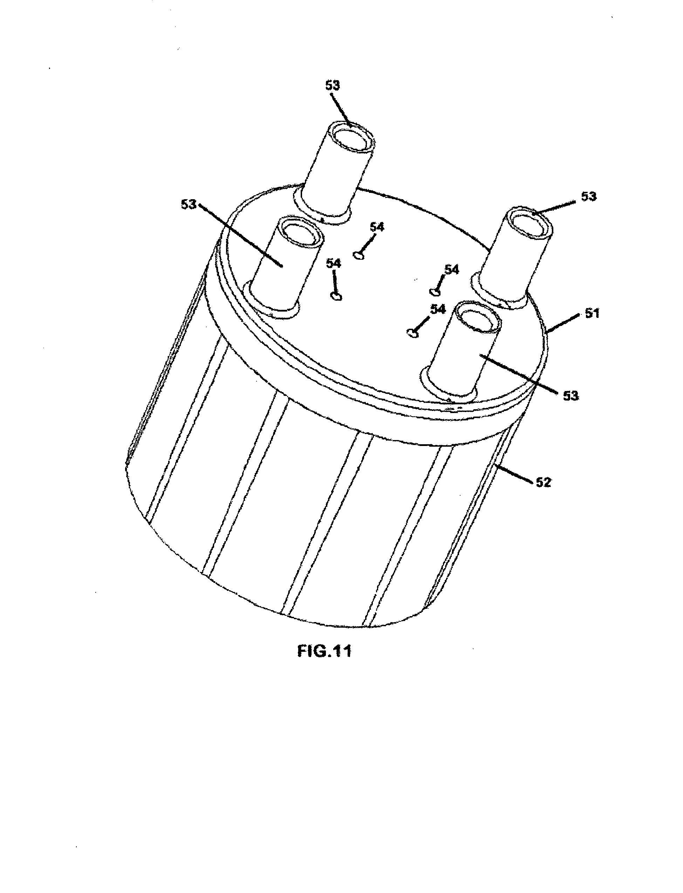

Figure 11 shows a third embodiment of the apparatus 50 according to the invention, comprising a housing with four inlets 53. The housing consists of a first housing element 51 and a second housing element 52, whereat four bores 54 are arranged in the first housing element 51 as vents. Both housing elements 51, 52 are designed cylindrically, whereat the inlets are arranged on a circumference line of the first housing element 51.

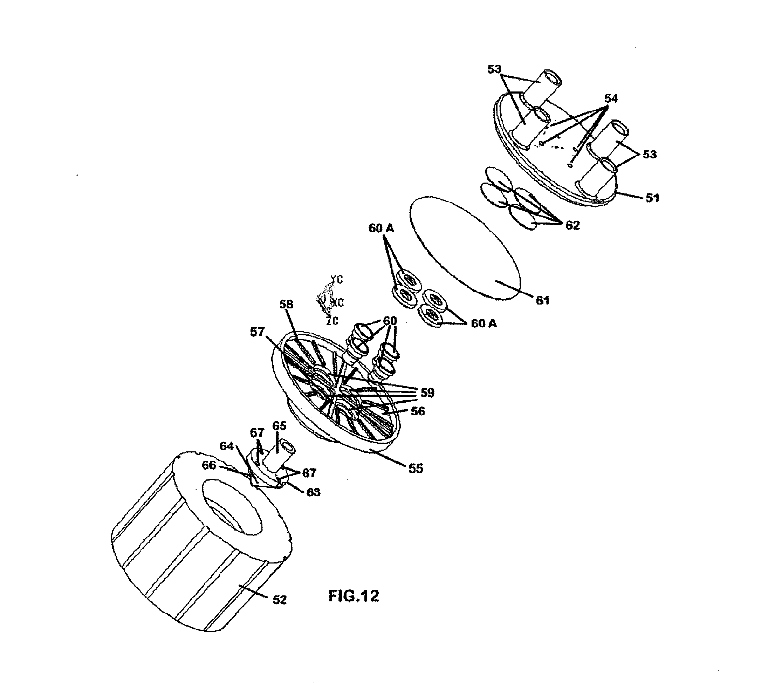

Figure 12 shows an exploded view of the third embodiment of the apparatus 50. The third embodiment comprises a third housing element 55 with a circular cross-section and a circumferential edge 56 extending over the surface facing the first housing element 51. Walls 57 extend from this inner surface, so that four regions are formed between the circumferential edge 56 and the walls 57. The third housing element 55 further features four recesses 59, which lead with one end to a respective region. A hydrophilic filter member 61 is arranged at the walls 57 and check valves 60 are arranged in the recesses 59. Discs 60 A are attached on the check valves by means of, e.g., welding. The discs provide each a protruding edge which comes to rest on a protruding edge of the recesses 59 and are attached to the third housing element by, e.g., welding. The entry chambers are formed in the first housing element 51 and are thus located upstream the hydrophilic filter member 61. The check valves 60 feature an overlapping edge which rests on the edges of the recesses 59. Furthermore, hydrophobic filters 62 are arranged at an inner surface of the first housing element 51 in such a way that they cover the bores 54. A fourth housing element with a conical section 64 and a cylindrical section 65 is arranged between the third housing element 55 and the second housing element 52. The conical outer surface of the conical section 64 rests on a likewise conically designed inner surface of the second housing element 52. In the conical area of the conical section 64 half channels 66 are formed, which, combined with the inner surface of the second housing element 52, each form a section of the flow path between an entry chamber and the collecting point of the apparatus. The conical section 64 of the fourth housing element 63 further features recesses 67, which communicate with a respective half channel 66. The recesses 67 also form a section of a flow path between an entry chamber and the collecting of the apparatus.

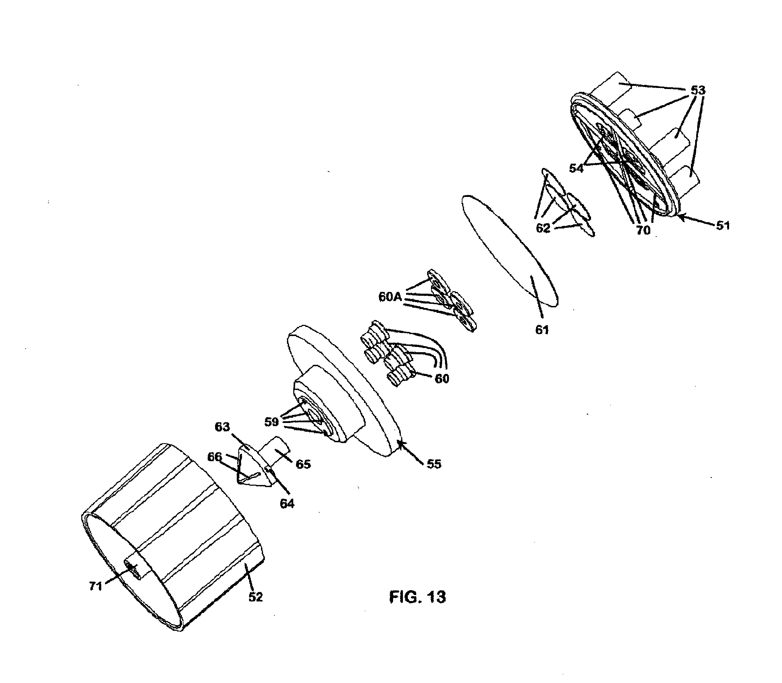

Figure 13 shows a further exploded view of the third embodiment of the apparatus 50 according to the invention. Figure 13 shows the bottom of the building elements of the third embodiment shown in figure 12. Walls 70 extend from an inner surface of the first housing element 51 facing the third housing element 55 and are arranged directly above the walls 57 of the third housing element 55. The hydrophilic filter member 61 arranged between the third housing element 55 and the first housing element 51 is trapped between the walls 70 and 57. Furthermore, it is connected to the circumferential edge 56 of the third housing element, e.g. by welding. The inner surface of the first housing element 51 facing the third housing element 51 further features the bores 54 as vents, which are covered by hydrophobic filters 62. The outlet 71 is formed on the second housing element 52. The walls 70 and the hydrophilic filter 61 border the entry chambers of the third embodiment

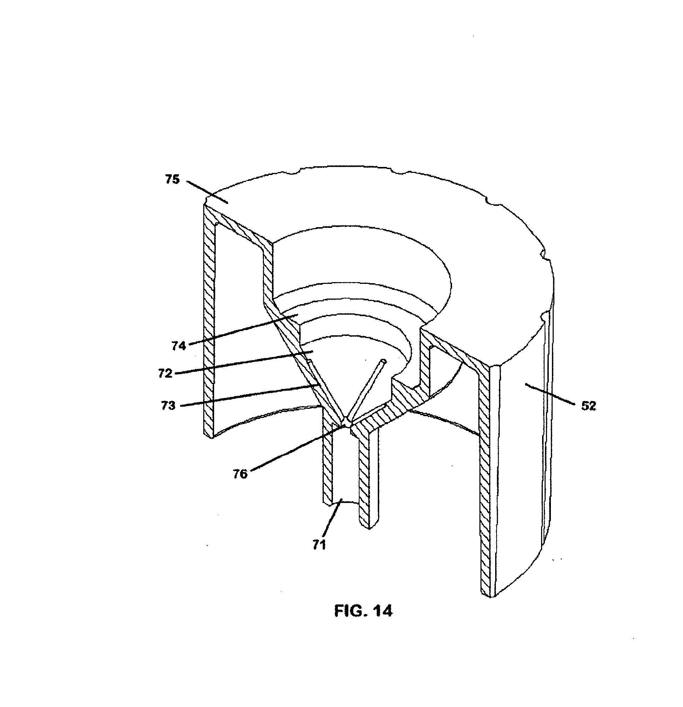

Figure 14 shows a sectional view of the second housing element 52. The outlet 71 is formed on the second housing element 52. The second housing element 52 features a conically designed inner surface 72 with half channels 73. The inner surface 72 rests on the conically formed area of the conical section 64 of the fourth housing element 63, whereat the half channels 66 of the conical section 64 are arranged directly above the half channels 73 of the inner surface 72. The half channels 73 form in combination with the half channels 66 a section of the flow path between the entry chambers and the collecting point 76. The channels formed by the half channels 66 and 73 lead to the collecting point 76, which is arranged upstream of the outlet 71. Due to the tactile contact between the inner surface 72 and the conical section 64 the channels formed by the half channels 73 and 66 are impermeably separated from each other. A step 74 is designed above the inner surface 72. The second housing element 52 furthermore features another inner surface 75. The third housing element 55 rests on both the step 74 and the inner surface 75, whereat the fourth housing element 63 is arranged between the third housing element 55 and the second housing element 52.

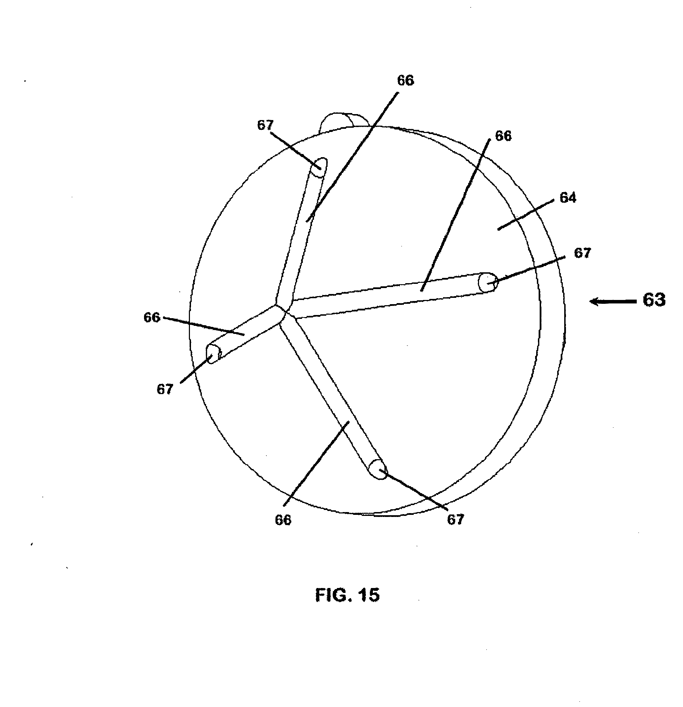

Figure 15 shows a perspective bottom view of the fourth housing element 63. Half channels 66 are formed on in the surface of the conical section 64 which lead into each other with the tapered end of the conical section 64 and communicate with a respective recess 67 with the other end. Furthermore, a nose 80 is formed on the conical section 64 of the fourth housing element 63, which rests in a corresponding recess of the second housing element 52, preventing the fourth housing element 63 within the apparatus from rotating.

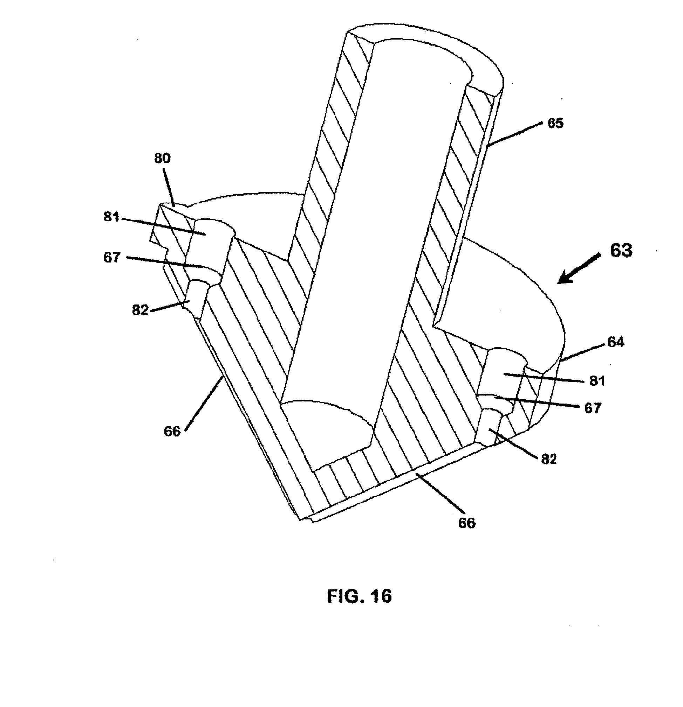

Figure 16 shows a sectional view of the fourth housing element 63, which features a cylindrical section 65 and a conical section 64. The fourth housing element 63 is designed as a hollow body. On the surface of the conical section 64 half channels 66 are formed, which are arranged above the half channels 72 of the inner surface 72 of the second housing element 52. The nose 80 formed on the conical section 64 prevents a rotation of the fourth housing element 63 towards the second housing element 52. The half channels 66 communicate with each other at the tip of the conical section 64 and lead to a respective recess 67 with their other ends. The recesses 67 are designed as a penetration member in this embodiment of the apparatus according to the invention, for the recesses 67 comprise a section 81 with a bigger cross-section area and a section 82 with a smaller cross-section area, which results in the cross-section area of the recesses 67 reducing in the direction of the stream.

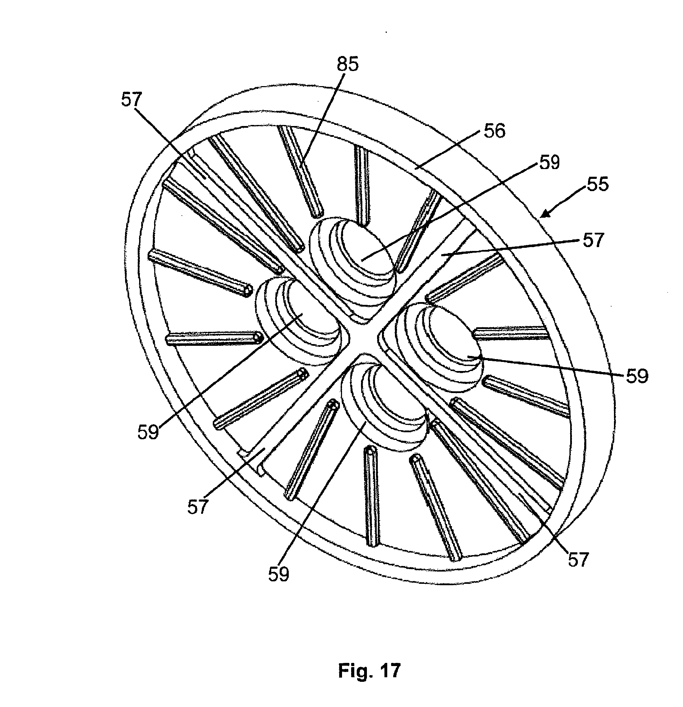

Figure 17 shows a perspective view of the third housing element 55. The third housing element features a circumferential edge 56 and walls 57, whereat the walls 57 extend from the inner surface of the third housing element 55 facing the first housing element 51. Furthermore, the third housing element 55 features recesses 59 which receive the check valves 60. Furthermore, ribs 85 extend from the inner surface of the third housing element 55 facing the first housing element in the regions between the walls 57. Those ribs 85 have an elongated form, and are in the regions between the walls 57 directed towards the recesses 59. The ribs 85 serve as support for the hydrophilic filter 61 and cause a planar flow of the fluids that have passed through the hydrophilic filter 61 in the direction of the respective recess 59. The hydrophilic filter 61 is arranged on the walls 57 and connected edgewise, e.g. welded, with the edge 56. The hydrophilic filter member 61 is trapped between the walls 57 of the third housing element 55 and the walls 70 of the first housing element 51.

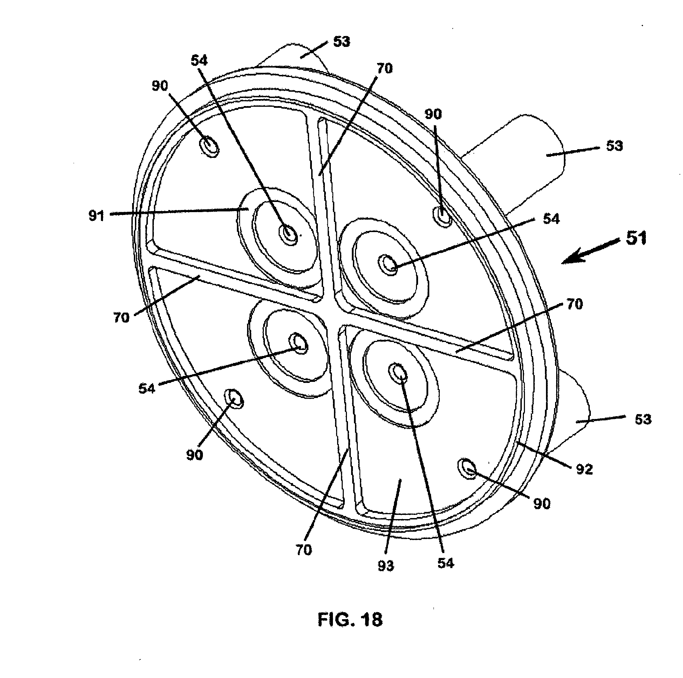

Figure 18 shows a perspective view of the first housing element 51 with four outlets 53, which communicate with the inside of the apparatus by recesses 90. The first housing element 51 furthermore features bores 54, which are surrounded by deepenings 91. Hydrophobic filters 62 are put into the deepenings 91. Gas bubbles contained in the fluids fed to the apparatus can escape through the bores 54 while the hydrophobic filters 62 prevent a simultaneous escape of the liquids through the gaps 54. Walls 70 extend from an inner surface 93 of the first housing element 51 facing the third housing element 55. Those walls 70 are connected to each other with one end and with the protruding edge 92 with the other end. The outer surface of the protruding edge 92 comes in tactile contact with the inner surface of the edge 56 of the third housing element 55. The regions between the walls 70 of the first housing element 51 and the hydrophilic filter member 61 form entry chambers which communicate with an inlet 53 respectively by a recess 90.

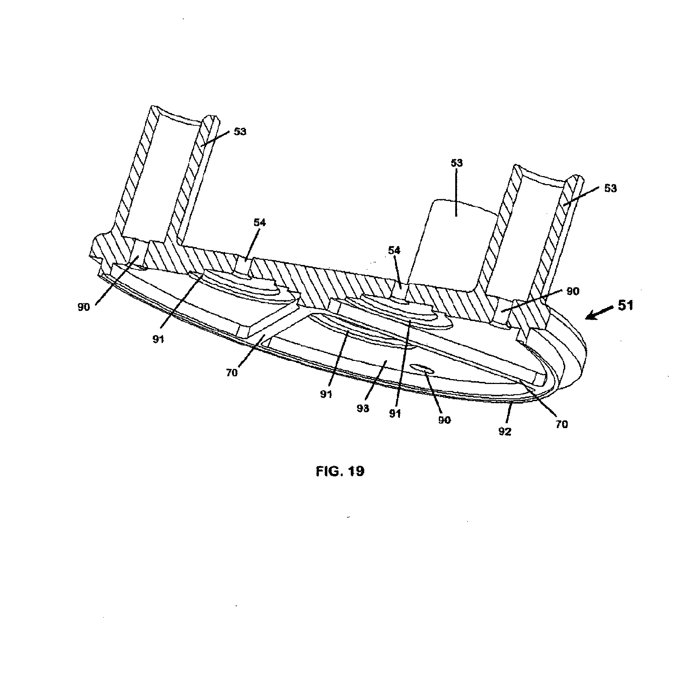

Figure 19 shows a sectional view of the first housing element 51. Each inlet 53 communicates with an entry chamber by a recess 90 respectively. The entry chambers are defined by the inner surface 93 of the first housing element 51, two respective walls 70 and the hydrophilic filter member 61. In the first housing element 51 further bores 54 are formed. The inner surface 92 features deepenings 91, which surround the bores 54 and into which the hydrophobic filters 62 are inserted. The recesses 90 have a smaller cross-section area than the inlets 53 and thereby causing a first storage of the fluids in the inlets 53.

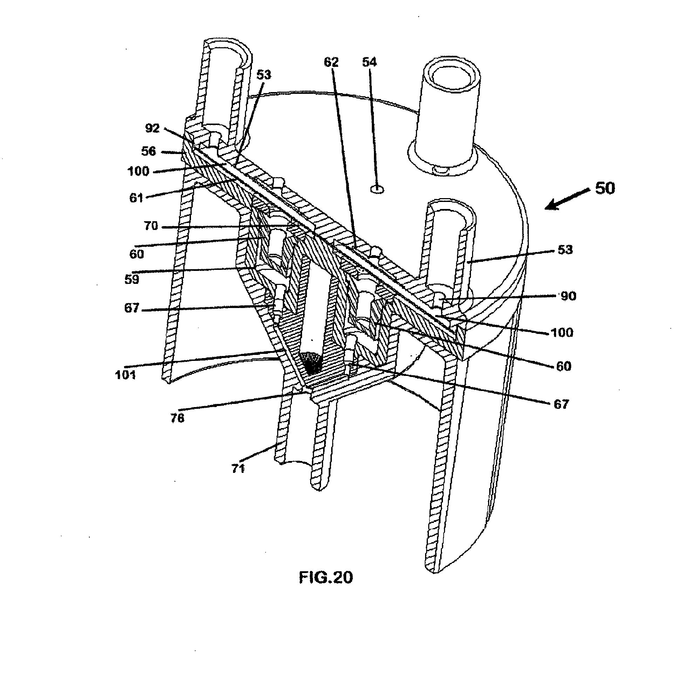

Figure 20 shows a sectional view of the third embodiment of the apparatus 50 according to the invention. In order to ensure a better overview merely some reference numbers are shown. The fluids fed to the apparatus 50 enter each entry chamber 100 through the inlets 53 and the recesses 90. The entry chambers 100 are bordered by the inner surface 93 of the first housing element, the protruding edge 92, the walls 70 extending from the inner surface 93, and the hydrophilic filter member 61. Correspondingly, the apparatus features four entry chambers 100. A flow path is respectively formed between the entry chambers 100 and the collecting point 76. The flow paths consist each of the regions between two walls 57 and the circumferential edge 56 of the third housing element 55, the recesses 59 of the third housing element 55, the recesses 67 and the channels 101, which are formed by the half channels 66 in the conical section 64 of the fourth housing element 63 as well as the half channels 73 in the inner surface 72 of the second housing element 52. In the recesses 59 of the third housing element 55 check valves 60 are arranged. The bores 54 in the first housing element 51 are covered by hydrophobic filters 62.

Once all fluids entering the entry chambers 100 through the inlets 53 have passed the hydrophilic filter member 61, they flow into the regions between the waals 57 and then into the direction of the recesses 59 of the third housing element 55, whereat the ribs formed on an the inner surfaces of the third housing element 55 both support the hydrophilic filter member 61 and cause a planar fluid flow towards the recesses 59. After having passed the check valves 60 the fluids enter the recesses 67 and flow through the channels 101 to the collection point 76, where they converge. Afterwards the converged fluids exit the apparatus 50 through the outlet 71. The inside of the apparatus 50 is designed with a dead volume that is as small as possible. Drop in pressure are caused both by the hydrophilic filter member 61 and the recesses 67, which are the penetration members of this embodiment. Further small drops in pressure are caused by the recesses 90 downstream the inlets 53 and by the end section with decreased cross-section area of the recesses 59, depending on their diameter. The hydrophilic filter member 61 causes downstream a drop in the pressure, since the fluids are merely able to flow through the pores of the hydrophilic filter member 61. The recesses 67 cause a drop in pressure at the collecting point 76, since the recesses 67 have a cross-section reducing in the direction of the flow.

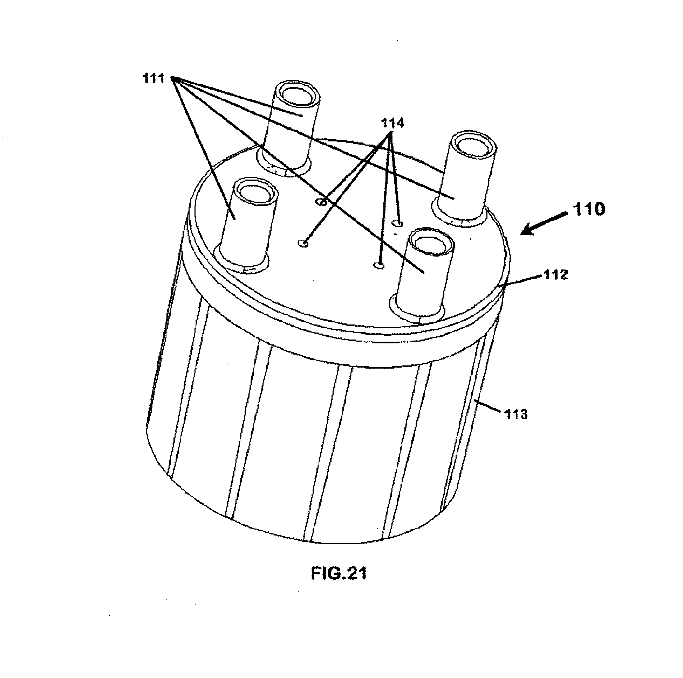

Figure 21 shows a fourth embodiment of an apparatus 110 according to the invention which is identical to the third embodiment 50 in its outward shape. The fourth embodiment features four inlets 111 which feed fluids to the apparatus 110. The apparatus 110 comprises a first housing element 112 and a second housing element 113 as well as a third housing element (not depicted) and a fourth housing element (not depicted). Four bores 114 are formed in the first housing element 112.

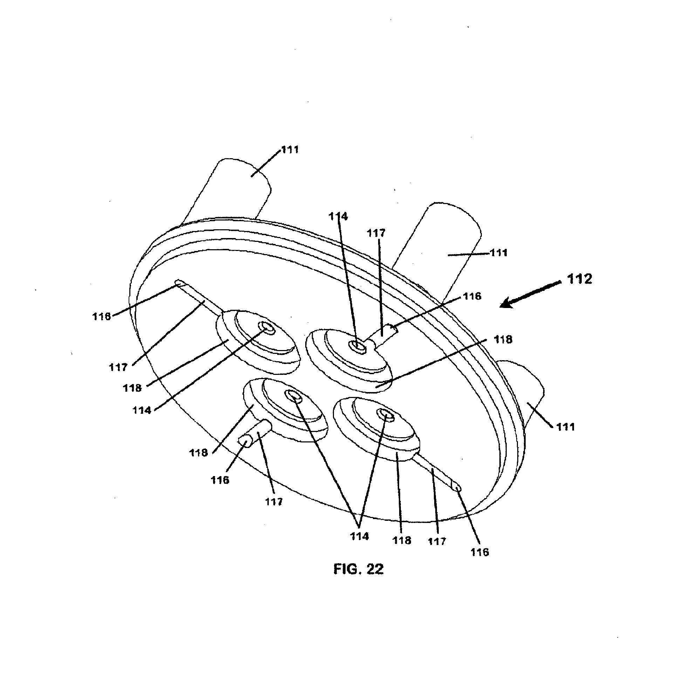

Figure 22 shows a perspective bottom view of the first housing element 112 of the fourth embodiment. The first housing element 112 features an inner surface 115 with recesses 116, which each communicate with an inlet 111. In the inner surface 115 half channels 117 and deepenings 118 are formed. The half channels 117 communicate with a respective recess 116 with the one end and with a deepening 118 with the other end. The deepenings 118 surround the bores 114, whereat hydrophobic filters are arranged in the deepenings 118. Therefore, gas bubbles can escape the fluids fed to the apparatus through the bores 114. The hydrophobic filters prevent fluids from also escaping the inside of the apparatus.

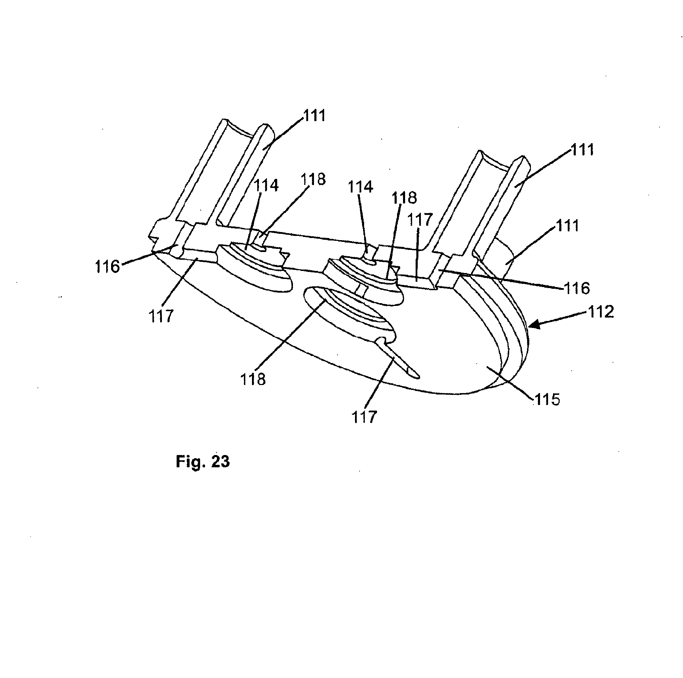

Figure 23 shows a cut view of the first housing element 112. Half channels 117, recesses 118, gaps 116 and gaps 114 are formed in the inner surface of the first housing element 112, whereat the gaps 116 are each connected with one half channel 117 and one inlet 11, and whereat the recesses 118 are arranged to surround each gap 114.

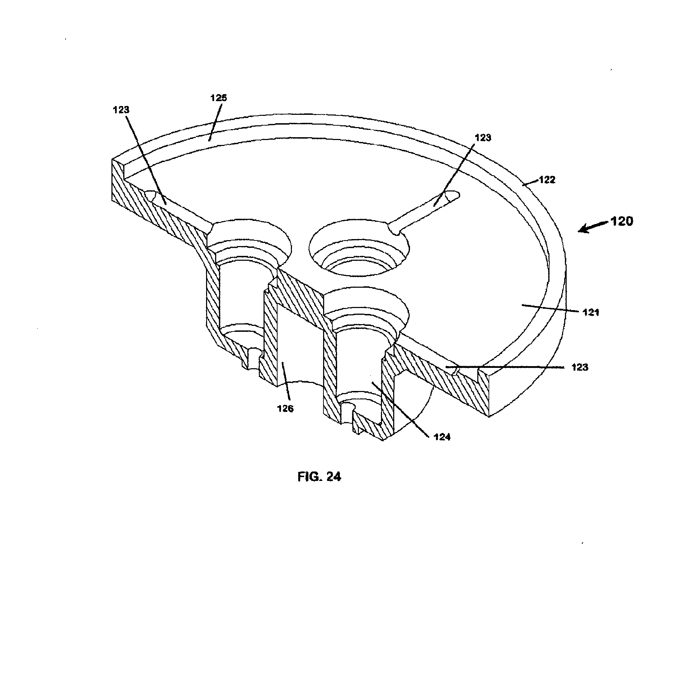

As the third embodiment of the apparatus according to the invention the fourth embodiment features a third housing element 120 and a fourth housing element, which is identical to the fourth housing element 63 of the third embodiment. The third housing element 120 of the fourth embodiment is shown in figure 24. The third housing element 120 is identical in its outward shape with the third housing element 55 of the third embodiment and features an inner surface 121 which faces the first housing element. Half channels 123 are formed in the inner surface 121. Furthermore, the third housing element 120 features recesses 124. The half channels 123 each lead to a recesses 124 with one end. The half channels 123 are arranged directly below the half channels 117 of the first housing element 112, so that the half channels 123 and 117 form channels when assembling the first housing element 112 and the third housing element 120. Those channels are each connected with a recess 116 of the first housing element 112 with one end and with a gap of the third housing element 120 with the other end. As in the third embodiment check valves are arranged in the recess 124, whereat the check valves are identical with the check valves 60 of the third embodiment. The inner surface 121 is bordered by a circumferential edge 122, whereat the inner lateral surface 125 of the circumferential edge 122 creates a frictional connection with the first housing element 121. Furthermore, a deepening 126 is formed in the third housing element 120, where the cylindrical section 65 of the fourth housing element 63 comes to rest.

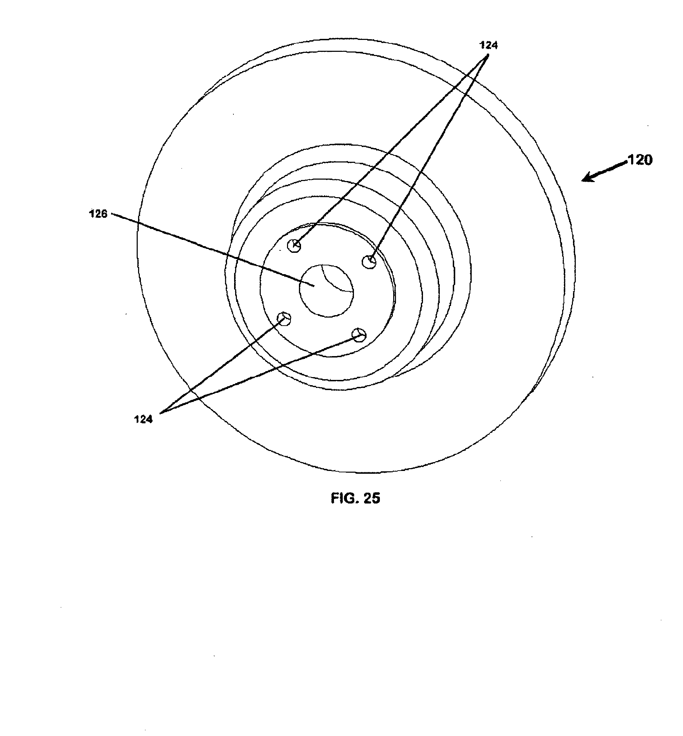

Figure 25 shows a perspective bottom view of the third housing element 120. The cylindrical section off the fourth housing element 63 is arranged in the deepening 126. The recesses 124 are connected to the half channels 123 formed in the inner surface 121.

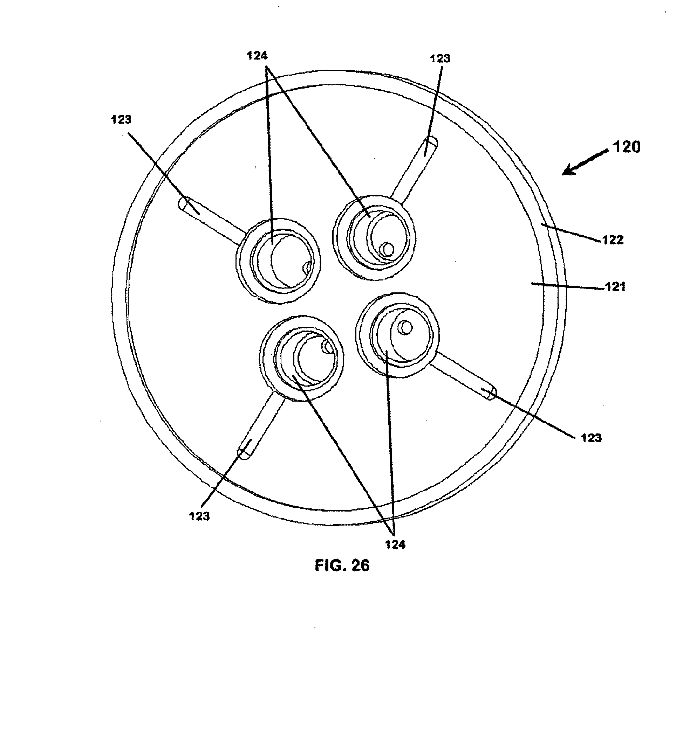

Figure 26 shows a perspective top view on the third housing element 121 of the fourth embodiment. The recesses 124 feature an end section with a reduced cross-section area.

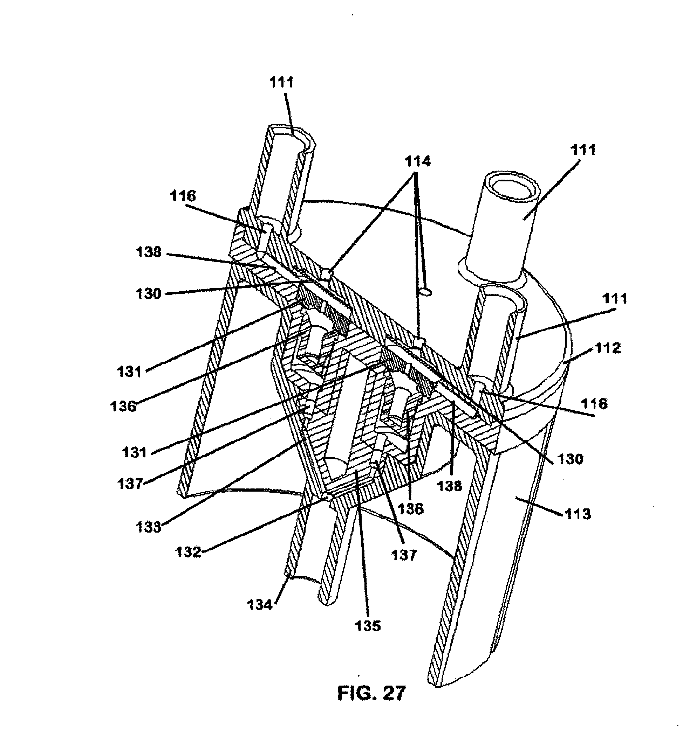

Figure 27 shows a sectional view of the fourth embodiment of the apparatus 110. The second housing element 113 is identical with the second housing element 52 of the third embodiment, and the fourth housing element 135 is identical with the fourth housing element 63 of the third embodiment. The check valves 136 are identical to the check valves 60 of the third embodiment. The fourth embodiment possesses two major differences to the third embodiment. First, the entry chambers 130 of the fourth embodiment are smaller than the entry chambers 100 of the third embodiment, since the inner surface 121 of the third housing element 120 comes to rest on the inner surface 115 of the first housing element 112 and forms channels 138 due to the half channels 117 in the inner surface 115 and the half channels in the inner surface 123. The recesses 116 of the first housing element 112 communicate with the entry chambers 130 by those channels 138. The second major difference is that there is no hydrophilic filter member arranged between the first housing element 112 and the third housing element 120. Instead, there are discs 131 with openings arranged in each recess 124 of the third housing element 120. The fluids, which have entered the entry chambers 130 through the inlets 111, flow through the openings in the discs 131 into the recesses 124 of the third housing element 120, in which check valves 136 are arranged. The small cross-section areas of the openings in the discs 131 cause a drop in pressure downstream of the discs 131. A further drop in pressure is caused by the recess 137 in the fourth housing element 135, since the cross-section area of the recesses 137 reduces in the direction of the flow. The recesses 137 lead to channels 133, which are formed by half channels in the conical surface of the fourth housing element 135 and in an inner surface of the second housing element 113. A fluid entering the apparatus through an inlet 111 flows through a recess 116 and a channel 138 into an entry chamber 130. The fluid then flows through the bore in the opening in a disc 131 and through a check valve into a recess 137 of the fourth housing element 135. The fluid subsequently flows through a channel 133 to the collecting point 132, at which the fluids that have entered through the inlets 111 converge. The converged fluids then exit the apparatus through the outlet 134.

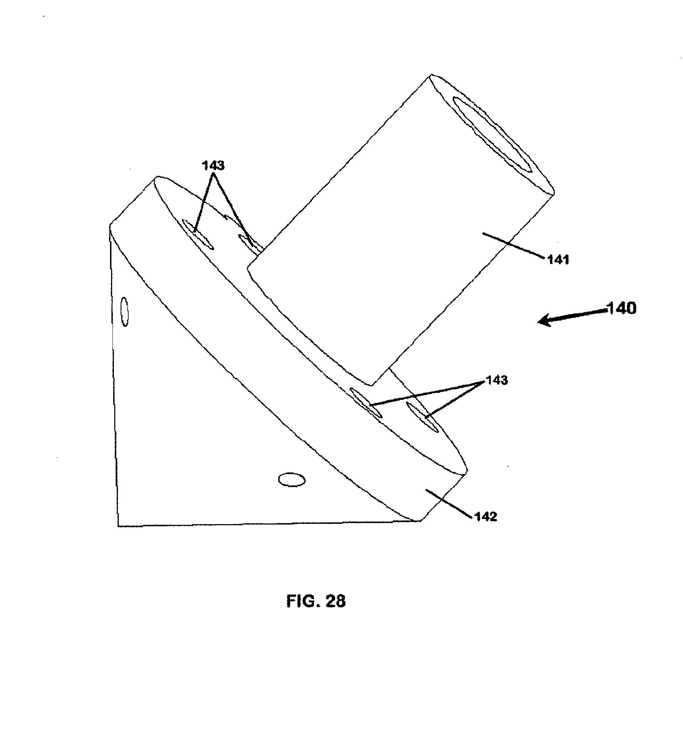

Figure 28 shows a second embodiment of the fourth housing element 140, comprising a cylindrical section 141 and a conical section 142. In contrast to the first embodiment of the fourth housing element 63 there are no half channels leading to the recesses 143 formed at the surface of the conical section 142. Instead the conical section 142 has a completely smooth surface 144.

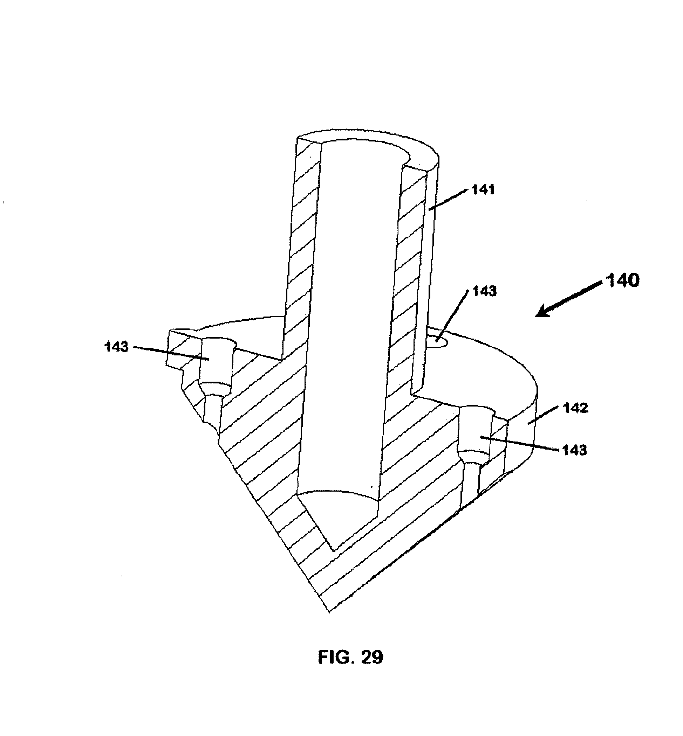

Figure 29 shows a sectional view of the second embodiment of the fourth housing element 140. The recesses 143 feature two sections with different cross-section areas, whereat the cross-section area of the recesses 143 are reduced in the direction of the flow.

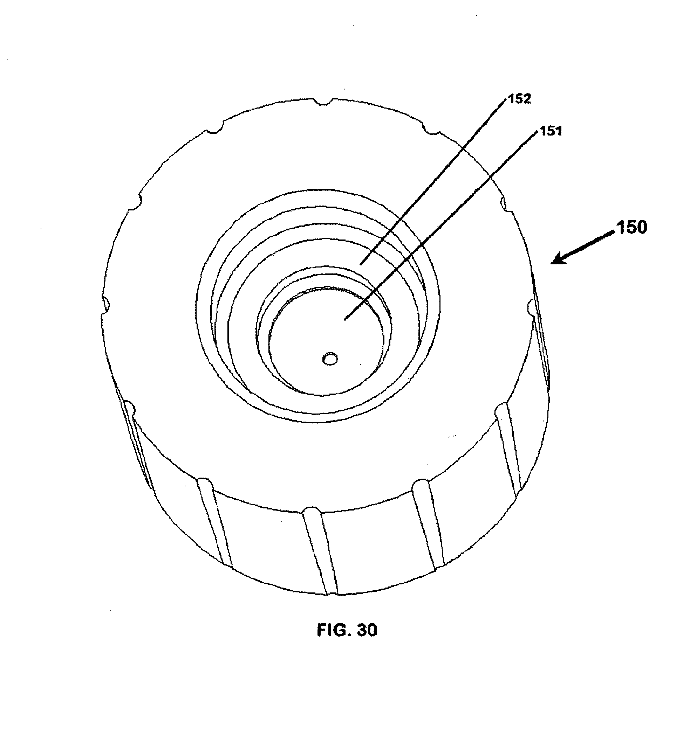

Figure 30 shows a second embodiment of the second housing element 150 with an inner surface 151, which is formed conically. A step 152 is formed above the inner surface 151 onto which a third housing element rests. There are no half channels formed in the inner surface 151, in contrast to the previous embodiments.

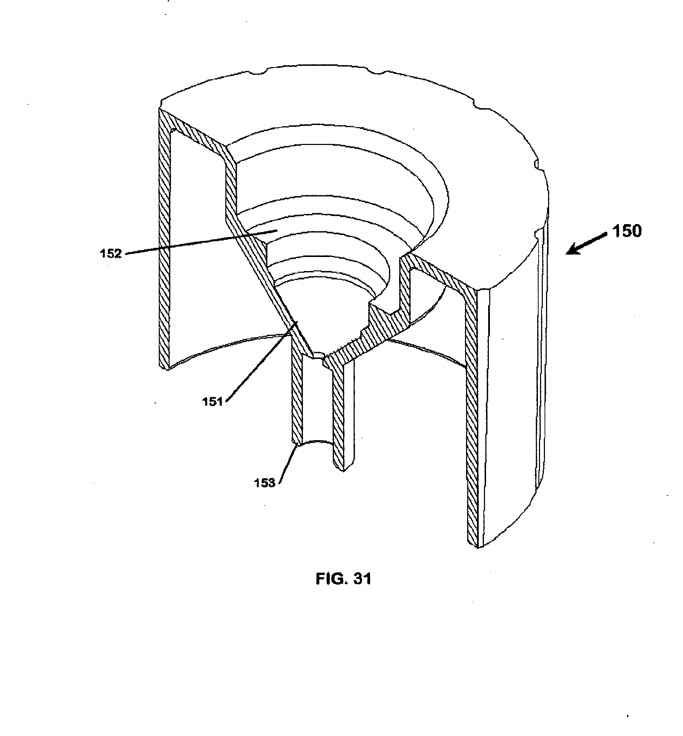

Figure 31 shows a sectional view of the second embodiment of the second housing element 150. An outlet 153 is formed on the second housing part 150. The inner surface 151 does not form any half channels, in contrast to the previous embodiments.

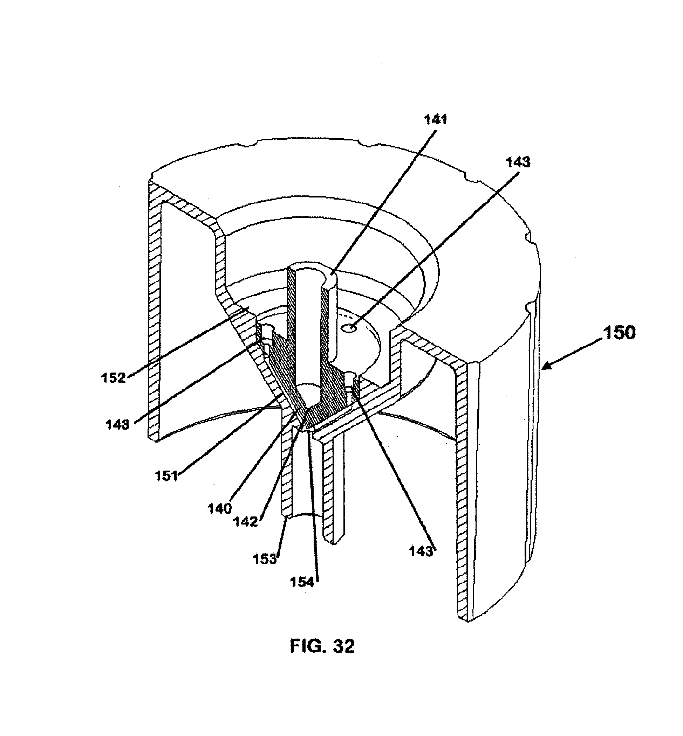

Figure 32 shows a sectional view of the second embodiment of the second housing element 150 and the fourth housing element 140. These two embodiments of the housing elements are intended to be a part of another embodiment of an apparatus according to the invention. In contrast to the previous embodiments, the fluids fed to the apparatus are combined in the area between the inner surface 151 of the second housing part 150 and the conically formed surface of the fourth housing element 140, for the conically formed surface of the conical section 142 and the inner surface 151 of the second housing element 150 do not touch, but form a conical region as a gap in which the fluids converge. The fluids flow from the conically formed area through a reduction 154 into the outlet 153. The collecting point is designed as a collecting chamber in this embodiment of the second housing element 150 and the fourth housing element 140, whereat the collecting chamber is the conically formed region between the inner surface 151 of the second housing element 150 and the conically formed surface of the conical section 142 of the fourth housing element.

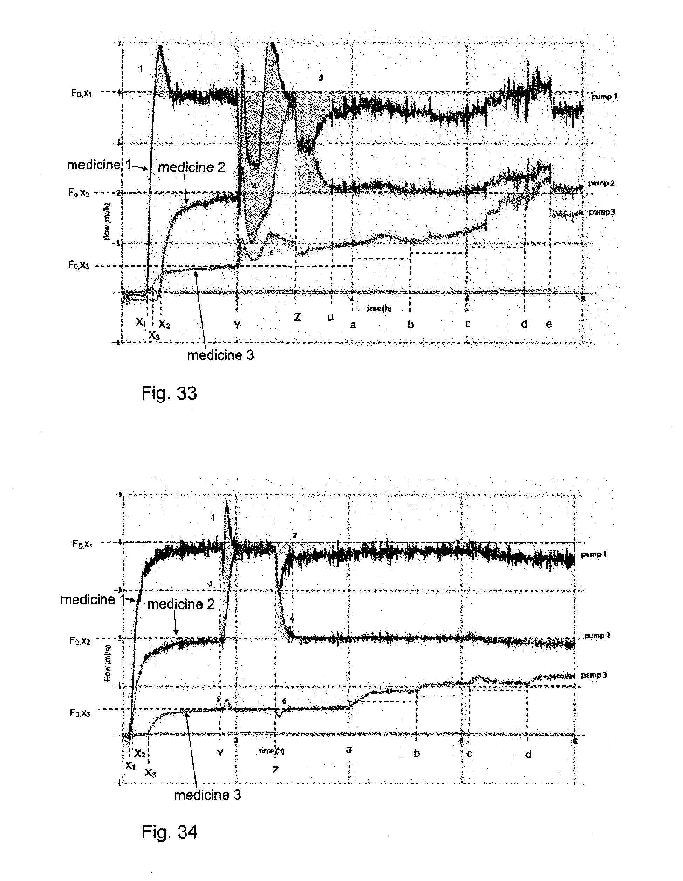

Figure 33 shows the result of a measurement of the flow rates of three medicines as a function of time, whereat the three medicines converge by means of an apparatus as known from prior art. The three pumps, which transport the medicines, are activated at the point of time t = 0. The three medicines have passed the apparatus at the points of time t = x₁, t = x₂ and t = x₃ and reach the measure point downstream of the apparatus. The flow rates of the medicines are now measured constantly. The flow rates set at the pumps are depicted in dashed lines and equal F_{0,x3} (0,5 ml/h) for medicine 3, F_{0,x2} (2,0 ml/h) for medicine 2 and F_{0,x1} (4,0 ml/h) for medicine 1. The measured flow rates are shown in complete lines. The flow rate of medicine 1 measured at the measure point initially exceeds the value set for F_{0,x1} at the pump. This overshoot is a first deviation of the flow rate set at the pump. After the overshoot, however, the flow rate of medicine 1 drops below the value set at the pump, which is a second deviation of the desired flow rate. The flow rate of medicine 2 slowly approaches the set value for F_{0,x2} without causing another overshoot. However, the flow rate of medicine 3 also surpasses the set value of F_{0,x3}. This deviation of the flow rate of medicine 3 is indeed caused by a calibration error and is not to be considered when converging medicines according to the prior art.

At the point of time t = y the flow rate of medicine 2 is set to the value of F_{0,x1} from the value of F_{0,x2} at the pump. As a result the flow rate of medicine 2 does not immediately increase to the set value, but fluctuates strongly and reaches the set value of F_{0,x1} only at the point of time t = z. In the meantime, between t = y and t = z, the actual flow rate of medicine 2 strongly deviates from the set value. Furthermore, the flow rates of the other two medicines 1 and 3 change in the timeframe of t =y and t = z as well, so that the dosages of those medicines are also imprecise. Both the flow rate of medicine 1 and the flow rate of medicine 3 fluctuate, even though their flow rates were not changed through pump settings. As a result all three medicines are incorrectly dosed when the flow rate of a single medicine is changed. This is the basic problem overcome by the apparatus for converging medicines according to the invention.

At the point of time t = z another change in the flow rate of medicine 2 by a setting of the pump occurs, whereat the flow rate is changed to the original value F_{0,x2}. The flow rate of medicine 2 only reaches the set value of F_{0,x2} at the point of time t = u, so that another imprecise dosage of medicine 2 occurs. The change of the flow rate of medicine 2 to the original value F_{0,x2} also leads to a change of the flow rate of medicine 1 and 3 at the measure point, so that the dosages of medicines 1 and 3 are imprecise as well.

At the points of time t = a, t = b, t = c and t = d the flow rate of medicine 3 was each increased by 0.1 ml per hour. In the sequence the flow rate of medicine 3 fluctuates at the measure point and increases in the time between t = c and t = e noticeably faster than the setting at the pump dictates. Consequently, an incremental change of the flow rate of a medicine also leads to an imprecise dosage of the medicine. Furthermore, the other medicines 1 and 2 also fluctuate noticeably in the time between t = a and t = e, which also results in an imprecise dosage of those medicines.

Figure 34 shows a measurement curve of flow rates as recorded by the apparatus according to the invention with the exact same changes. The pumps are activated at the point of time t = 0, and the medicines reach the measure point at t = x₁, t = x₂ and t = x₃. The measure point is situated downstream of the apparatus according to the invention. At t = y the flow rate of medicine 2 is raised from its original value F_{0,x2} (2,0 ml/h) to a value of F_{0,x1} (4,0 ml/h) by a setting of the pump and reduced from the value F_{0,x1} to the value of F_{0,x2} at t=z. At the points of time t = a, t = b, t = c, and t = d the flow rate of medicine 3 is increased by 0.1 ml per hour starting from the value F_{0,x3} (0,5 ml/h). The flow rates of the medicines do not show an overshoot, which is a first huge advantage of the apparatus according to the invention. Furthermore, the flow rate of medicine 2 quickly approaches the set value of F_{0,x1} after changing it without any fluctuations. Also, the flow rate of medicine 2 quickly approaches the value F_{0,x2} after reducing the flow rate of medicine 2 at t = z. A change of the set flow rate of medicine 2 does change the flow rates of the other medicines at the measure point, but in the form of a peak with a very narrow half width, so that the resulting imprecise dosage of medicines 1 and 3 are minimal. The measure curves of the flow rates of medicines 1 and 3 have narrow peaks both in the immediate proximity of the point of time t = y and in the immediate proximity of t = z. This short-lived influence of the other flow rates when manipulating one flow rate is a second advantage of the apparatus according to the invention. The incremental change of the set flow rates of medicine 3 at the times t = a, t = b, t = c and t = d, however, does not lead to a measurable change in the flow rates of medicines 1 and 2 at the measure point. The independence of the other measured flow rates of an incremental change of one flow rate is a third major advantage of the apparatus according to the invention.

The laboratory tests shown in Figures 33 and 34 show the differences between a infusion set of prior art and the apparatus according to the invention. A clinical situation is simulated, whereat the flow rate of medicine 2 is changed suddenly from 2.0 ml/h to 4.0 ml/h and the flow rate of medicine 3 is changed gradually from 0.5 ml/h to 1.0 ml/h. The Results show improvements in stability of flow rates, less deviations after a change of a flow rate and shorter start-up time.

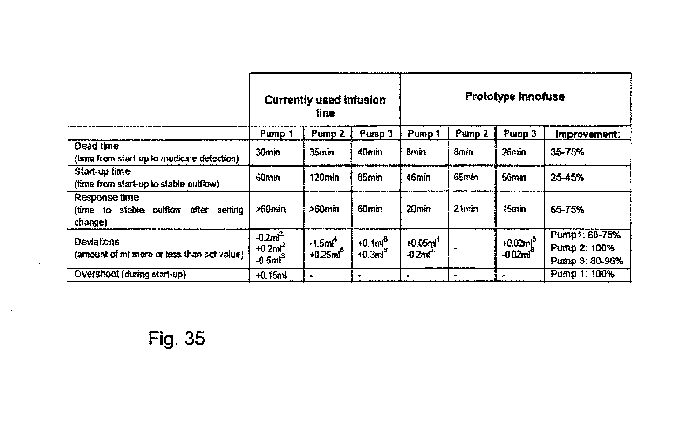

Figure 35 shows a table summarizing the basic advantages of the apparatus according to the invention in comparison to an apparatus as known from prior art. The dead time is the time between starting of the pumps and first detection of a medicine at the measure point. The start-up time is the time between starting the pumps until a constant outflow is reached after a change in pump settings. The response time is the time until a constant outflow is reached after a change in the pump settings. An overshoot is defined to be the excessive amount of medicine that is administered compared to the set value during a limited time frame, following the start up of the pumps or a flow rate change. An underhoot is defined to be the amount of medicine that is to less administered compared to the set value during a limited time frame, following the start up of the pumps or a flow rate change. The accuracy is the ratio of administered volume of a particular medicine to the volume according to the set pump value.

## Claims

1. Apparatus for simultaneous multiple medicament administration comprising a housing, a plurality of inlets and one outlet
**characterized in that**
at least two entry chambers and a collecting point are formed within the housing which consists of a stiff material, wherein each entry chamber communicates with one inlet and is separated from the other entry chambers impervious to fluids and wherein the collecting point communicates with the outlet
and **in that**
the entry chambers communicate with the collecting point respectively via a flow path in which at least one penetration member is arranged causing a decrease in pressure downstream the penetration member with respect to the pressure upstream the penetration member.

2. Apparatus according to claim 1
**characterized in that**
the penetration member is a filter member, a membrane, an elastic member comprising at least one slit, a wall with at least one opening, a channel with a portion of decreased cross section area or an adjustable valve.

3. Apparatus according to claim 1 or 2,
**characterized in that**
two or more penetration members are arranged consecutively within one or more flow paths respectively such that a fluid flowing through a flow path passes one penetration member after another.

4. Apparatus according to claim 1, 2 or 3
**characterized in that**
the housing consists of a stiff polymeric material or a stiff polymeric material, which is transparent for visible light.

5. Apparatus according to at least one of the claims 1 to 4
**characterized in that**
one or more flow paths are formed as flow channels and/or
the collecting point is formed as a collecting chamber.

6. Apparatus according to at least one of the claims 1 to 5
**characterized in that**
at least one penetration member is a filter member which consists of two or more filter elements, wherein each filter element is arranged in one flow path respectively and /or
two or more filter elements consist of different materials and/or two or more filter elements have different meshes, pore sizes, electrical charges and/or the filter member is provided with an additional antibacterial substance or the filter elements are provided with the same or different additional antibacterial substances.

7. Apparatus according to claim 6
**characterized in that**
the outlet is positioned on the housing centrically in case of equal filter element surfaces or any other position in case of unequal filter element surfaces.

8. Apparatus according to at least one of the claims 1 to 7
**characterized in that**
at least one penetration member is a filter member which is preassembled in a filter frame by joining, molding, glueing, ultrasonic welding or heat sealing, wherein the filter frame is fixed on at least one inner surface of the housing.

9. Apparatus according to at least one of the claims 1 to 8
**characterized in that**
the entry chambers are separated from each other by walls protruding from at least one inner surface of the housing and/or by walls protruding from at least one penetration member arranged within one or more entry chambers or in the vicinity thereof and/or
the housing comprises ribs which protrude from at least one inner surface of the housing, which ribs providing a planar fluid stream of fluids within the entry chambers respectively.

10. Apparatus according to at least one of the claims 1 to 9
**characterized in that**
one or more entry chambers communicate with one inlet respectively via a flow channel with a decreased cross section area with respect to the cross section area of the inlet.

11. Apparatus according to at least one of the claims 1 to 10
**characterized in that**
a check valve is positioned within at least one flow paths, within at least one entry chamber or within at least one inlet and/or
at least one bore is formed on the housing, wherein the bore is covered by a hydrophobic filter, thereby allowing for gas bubbles in the fluids fed to the entry chambers to escape from the fluids.

12. Apparatus according to at least one of the claims 1 to 11
**characterized in that**
at least one penetration member is fixed on an inner surface of the housing by joining, molding, glueing, ultrasonic welding or heat sealing.

13. Apparatus according to at least one of the claims 1 to 12
**characterized in that**
the inlets are positioned on a circumference line of the housing, which circumference line has a diameter of 1 mm to 40 mm and/or
the collecting chamber has an internal volume of 0.1 ml to 1 ml.

14. Apparatus according to at least one of the claims 1 to 13
**characterized in that**
at least one inlet is rotatable and/or
the housing consists of one or more housing elements, wherein the housing elements are welded, press fitted, glued and/or snap fitted with each other.

15. Method for simultaneous multiple medicament administration, wherein the medicaments are fluids
**characterized in that**
the fluids are fed to a collecting point via separate flow paths and at least one penetration member is arranged within each flow path causing a decrease in pressure downstream the penetration member with respect to the pressure upstream the penetration member, wherein the fluids converge at the collecting point.

16. Method according to claim 15
**characterized in that**
the penetration member is a filter member, a membrane, an elastic member comprising at least one slit, a wall with at least one opening or a channel with a portion of decreased cross section area and/or
one or-more penetration members are arranged consecutively within one or more flow paths such that a fluid flowing through a flow path passes one penetration member after another.

## Amended claims

### Amended claims in accordance with Rule 137(2) EPC.

**1.** Apparatus (1, 50, 110) for simultaneous multiple medicament administration of fluid medicaments with changing flow rates, comprising a housing (2) consisting of a stiff material, a plurality of inlets (3, 53, 111) and one outlet (5, 71), wherein at least two entry chambers (9, 21, 100, 130) communicating with one inlet (5, 71) respectively and being separated from each other impervious to fluids and a collecting point (22, 44, 76) communicating with the outlet (5, 71) are formed within the housing (2),
**characterized in that**
the entry chambers (9, 21, 100, 130) communicate with the collecting point (22, 44, 76) respectively via a flow path in which at least one penetration member (14, 45, 61, 101, 133) is arranged causing a decrease in pressure downstream the penetration member (14, 45, 61, 101, 133) with respect to the pressure upstream the penetration member (14, 45, 61, 101, 133), such that the pressure in the collecting point (22, 44, 76) is lower than the pressure in each inlet (3, 53, 111), wherein at least one of the penetration members_(14, 45, 61, 101, 133) arranged in each flow path is a filter member (14, 61) and/or a channel with a portion of decreased cross section area (45, 101, 133).

**2.** Apparatus (1, 50, 110) according to claim 1,
**characterized in that**
two or more penetration members (14, 45, 61, 101, 133) are arranged consecutively within one or more flow paths respectively such that a fluid flowing through a flow path passes one penetration member (14, 45, 61, 101, 133) after another.

**3.** Apparatus (1, 50, 110) according to claim 1 or 2
**characterized in that**
the housing (2) consists of a stiff polymeric material or a stiff polymeric material, which is transparent for visible light.

**4.** Apparatus (1, 50, 110) according to at least one of the claims 1 to 3
**characterized in that**
one or more flow paths are formed as flow channels and/or
the collecting point (22, 44, 76) is formed as a collecting chamber.

**5.** Apparatus (1, 50, 110) according to at least one of the claims 1 to 4
**characterized in that**
at least one penetration member (14, 45, 61, 101, 133) is a filter member (14, 61) which consists of two or more filter elements, wherein each filter element is arranged in one flow path respectively and /or two or more filter elements consist of different materials and/or two or more filter elements have different meshes, pore sizes, electrical charges and/or the filter member is provided with an additional antibacterial substance or the filter elements are provided with the same or different additional antibacterial substances.

**6.** Apparatus (1, 50, 110) according to claim 5
**characterized in that**
the outlet is positioned on the housing (2) centrically in case of equal filter element surfaces or any other position in case of unequal filter element surfaces.

**7.** Apparatus (1, 50, 110) according to at least one of the claims 1 to 6
**characterized in that**
at least one penetration member (14, 45, 61, 101, 133) is a filter member (14, 61) which is preassembled in a filter frame by joining, molding, glueing, ultrasonic welding or heat sealing, wherein the filter frame is fixed on at least one inner surface of the housing.

**8.** Apparatus (1, 50, 110) according to at least one of the claims 1 to 7
**characterized in that**
the entry chambers (9, 21, 100, 130) are separated from each other by walls (10, 20, 43, 57, 70) protruding from at least one inner surface of the housing (2) and/or by walls protruding from at least one penetration member arranged within one or more entry chambers or in the vicinity thereof and/or
the housing (2) comprises ribs (24, 85) which protrude from at least one inner surface of the housing (2), which ribs (24, 85) providing a planar fluid stream of fluids within the entry chambers (9, 21, 100, 130) respectively.

**9.** Apparatus (1, 50, 110) according to at least one of the claims 1 to 8
**characterized in that**
one or more entry chambers (9, 21, 100, 130) communicate with one inlet respectively via a flow channel with a decreased cross section area with respect to the cross section area of the inlet.

**10.** Apparatus (1, 50, 110) according to at least one of the claims 1 to 9
**characterized in that**
a check valve (13, 60, 136) is positioned within at least one flow paths, within at least one entry chamber (9, 21, 100, 103) or within at least one inlet (3, 53, 111) and/or
at least one bore (11, 54, 114) is formed on the housing (2), wherein the bore (11, 54, 114) is covered by a hydrophobic filter (62), thereby allowing for gas bubbles in the fluids fed to the entry chambers (9, 21, 100, 130) to escape from the fluids.

**11.** Apparatus (1, 50, 110) according to at least one of the claims 1 to 10
**characterized in that**
at least one penetration member (14, 45, 61, 101, 133) is fixed on an inner surface of the housing (2) by joining, molding, glueing, ultrasonic welding or heat sealing.

**12.** Apparatus (1, 50, 110) according to at least one of the claims 1 to 11
**characterized in that**
the inlets (3, 53, 111) are positioned on a circumference line of the housing (2), which circumference line has a diameter of 1 mm to 40 mm and/or
the collecting chamber (22, 44, 76) has an internal volume of 0.1 ml to 1 ml.

**13.** Apparatus (1, 50, 110) according to at least one of the claims 1 to 12
**characterized in that**
at least one inlet (3, 53, 111) is rotatable and/or
the housing (2) consists of one or more housing elements (6, 7, 40, 51, 52, 55, 63, 112, 113, 120, 140, 150), wherein the housing elements (6, 7, 40, 51, 52, 55, 63, 112, 113, 120, 140, 150) are welded, press fitted, glued and/or snap fitted with each other.

**14.** Method for simultaneous multiple medicament administration of fluid medica-ments with changing flow rates, which are fed to a collecting point (22, 44, 76) via separate flow paths and which converge at the collecting point (22, 44, 76),
**characterized in that**
at least one penetration member (14, 45, 61, 101, 133) is arranged within each flow path causing a decrease in pressure downstream the penetration member (14, 45, 61, 101, 133) with respect to the pressure upstream the penetration member (14, 45, 61, 101, 133), such that the pressure in the collecting point (22, 44, 76) is lower than the pressure in each inlet (3, 53, 111), wherein at least one of the penetration members (14, 45, 61, 101, 133) arranged in each flow path is a filter member (14, 61) and/or a channel with a portion of decreased cross section area (45, 101, 133).

**15.** Method according to claim 14
**characterized in that**
the penetration member (14, 45, 61, 101, 133) is a filter member (14, 61) or a channel with a portion of decreased cross section area and/or one or more penetration members (14, 45, 61, 101, 133) are arranged consecutively within one or more flow paths such that a fluid flowing through a flow path passes one penetration member (14, 45, 61, 101, 133) after another.
